(19) Europäisches Patentamt
European Patent Office
Office européen des brevets

(11) Veröffentlichungsnummer: **0 395 582 A2**

(12) # EUROPÄISCHE PATENTANMELDUNG

(21) Anmeldenummer: **90810307.0**

(22) Anmeldetag: **18.04.90**

(51) Int. Cl.[5]: **C07C 271/62, C07C 217/20, A01N 47/10**

(30) Priorität: **27.04.89 CH 1599/89**

(43) Veröffentlichungstag der Anmeldung: **31.10.90 Patentblatt 90/44**

(84) Benannte Vertragsstaaten: **AT BE CH DE DK ES FR GB IT LI NL**

(71) Anmelder: **CIBA-GEIGY AG**
**Klybeckstrasse 141**
**CH-4002 Basel(CH)**

(72) Erfinder: **Karrer, Friedrich, Dr.**
**Rebbergstrasse 5**
**CH-4800 Zofingen(CH)**

(54) **Bisacyläthylamine.**

(57) Neue N-acylierte 2-[4-(Halogenphenoxy)-phenoxy]-äthylcarbaminsäureester der Formel I

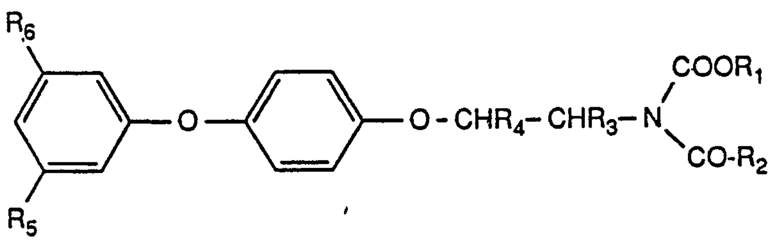

(I)

worin
$R_1$ $C_1$-$C_8$-Alkyl oder $C_3$-$C_5$-Alkenyl,
$R_2$ $C_1$-$C_8$-Alkyl, $C_1$-$C_8$-Alkoxy, -CO-$R_7$ oder -$NR_8R_9$,
$R_3$ und $R_4$ unabhängig voneinander für Wasserstoff oder Methyl,
$R_5$ für Chlor oder Fluor,
$R_6$ entweder für den gleichen Substituenten wie $R_5$ oder für Wasserstoff,
$R_7$ $C_1$-$C_8$-Alkoxy oder -$NR_{10}R_{11}$,
$R_8$ für $C_1$-$C_4$-Alkyl,
$R_9$ für $C_1$-$C_4$-Alkyl oder
$R_8$ und $R_9$ zusammen für eine $C_4$-$C_6$-Alkylenkette, welche durch Sauerstoff, Schwefel oder -$NCH_3$- unterbrochen sein kann,
$R_{10}$ für Wasserstoff oder $C_1$-$C_4$-Alkyl und
$R_{11}$ für Wasserstoff oder $C_1$-$C_4$-Alkyl, Benzyl, Phenyl oder durch Halogen oder Methyl substituiertes Phenyl oder
$R_{10}$ und $R_{11}$ zusammen für eine $C_4$-$C_6$-Alkylenkette, welche durch Sauerstoff, Schwefel oder -$NCH_3$- unterbrochen sein kann,
stehen; ihre Herstellung, ihre Verwendung in der Schädlingsbekämpfung sowie Schädlingsbekämpfungsmittel, welche diese Carbaminsäureester als Wirkstoff enthalten, werden offenbart. Bevorzugtes Anwendungsgebiet ist die Bekämpfung von Schädlingen an Tieren und Pflanzen, insbesondere von Eiern und Larven phytophager Schadinsekten und -milben.

EP 0 395 582 A2

## Bisacyläthylamine

Die vorliegende Erfindung betrifft neue N-acylierte 2-[4-(Halogenphenoxy)-phenoxy]-äthylcarbaminsäureester, ihre Herstellung, ihre Verwendung in der Schädlingsbekämpfung sowie Schädlingsbekämpfungsmittel, welche diese Carbaminsäureester als Wirkstoff enthalten.

Die erfindungsgemässen N-Acyl-2-[4-(halogenphenoxy)-phenoxy]-äthylcarbaminsäureester entsprechen der Formel I

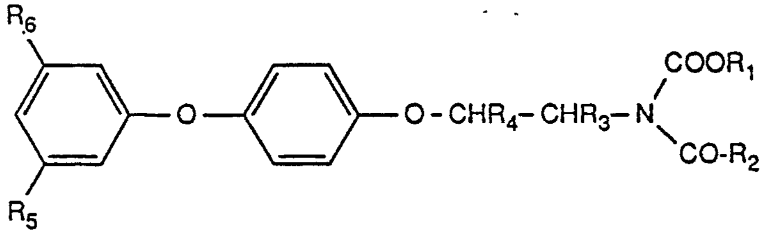

(I)

worin

$R_1$ $C_1$-$C_8$-Alkyl oder $C_3$-$C_5$-Alkenyl,

$R_2$ $C_1$-$C_8$-Alkyl, $C_1$-$C_8$-Alkoxy, -CO-$R_7$ oder -$NR_8R_9$,

$R_3$ und $R_4$ unabhängig voneinander für Wasserstoff oder Methyl,

$R_5$ für Chlor oder Fluor,

$R_6$ entweder für den gleichen Substituenten wie $R_5$ oder für Wasserstoff,

$R_7$ $C_1$-$C_8$-Alkoxy oder -$NR_{10}R_{11}$, $R_8$ für $C_1$-$C_4$-Alkyl,

$R_9$ für $C_1$-$C_4$-Alkyl oder

$R_8$ und $R_9$ zusammen für eine $C_4$-$C_6$-Alkylenkette, welche durch Sauerstoff, Schwefel oder -$NCH_3$- unterbrochen sein kann,

$R_{10}$ für Wasserstoff oder $C_1$-$C_4$-Alkyl und

$R_{11}$ für Wasserstoff oder $C_1$-$C_4$-Alkyl, Benzyl, Phenyl oder durch Halogen oder Methyl substituiertes Phenyl oder

$R_{10}$ und $R_{11}$ zusammen für eine $C_4$-$C_6$-Alkylenkette, welche durch Sauerstoff, Schwefel oder -$NCH_3$- unterbrochen sein kann,

stehen.

Unter Halogen sind in der Definition von $R_{11}$ Fluor, Chlor, Brom oder Jod zu verstehen, vorzugsweise aber Chlor.

$C_1$-$C_8$-Alkylgruppen können geradkettig oder verzweigt sein. Als Beispiele solcher Reste seien Methyl, Aethyl, Propyl, Isopropyl oder Butyl und seine Isomeren sowie die möglichen Strukturisomeren der $C_5$-$C_8$-Alkyle genannt. Bevorzugte Alkylgruppen enthalten höchstens 4 Kohlenstoffatome. Besonders bevorzugt sind Methyl und Aethyl.

$C_3$-$C_5$-Alkenylgruppen der Definition von $R_1$ können geradkettig oder verzweigt sein. Die Doppelbindung dieser Gruppen ist von der Bindung zum Sauerstoffatom stets durch ein gesättigtes Kohlenstoffatom getrennt. Beispiele sind Allyl, Methallyl, 2-Butenyl, 3-Butenyl, 2-Pentenyl, 3-Pentenyl oder 4-Pentenyl.

$C_1$-$C_8$-Alkoxyreste der Definition von $R_2$ und $R_7$ sind im Rahmen vorliegender Erfindung Methoxy, Aethoxy, Propoxy, Isopropoxy oder die vier isomeren Butoxy oder die möglichen Strukturisomeren der $C_5$-$C_8$-Alkoxyreste. Hervorgehoben werden die kurzkettigen Alkoxygruppen mit weniger als 5 Kohlenstoffatomen. Bevorzugt sind hiervon Methoxy und Aethoxy.

Falls der Rest $R_6$ eine von Wasserstoff abweichende Bedeutung hat, also für Fluor oder Chlor steht, steht im Rahmen der Erfindung $R_6$ dann für das gleiche Halogenatom wie $R_5$.

Falls $R_8$ und $R_9$, oder $R_{10}$ und $R_{11}$ gemeinsam eine gegebenenfalls durch Sauerstoff, Schwefel oder -$NCH_3$- unterbrochene $C_4$-$C_6$-Alkylenkette bedeuten, wird von den Gruppen -$NR_8R_9$ oder -$NR_{10}R_{11}$ ein über das Stickstoffatom gebundener Heterocyclus gebildet. Diesen Heterocyclen liegen die Strukturen von beispielsweise Pyrrolidin, Piperidin, Perhydroazepin, Oxazolidin, Thiazolidin, Imidazolidin, Pyrazolidin, Perhydropyrimidin, Morpholin, Thiomorpholin, Perhydropyridazin, Isoxazolidin, Isothiazolidin oder Piperazin zugrunde.

Pestizide Aethylcarbaminsäure-Derivate sind aus der Literatur schon verschiedentlich bekannt geworden, jedoch befriedigen diese Substanzen bezüglich des erzielten Wirkungsspektrums nicht voll oder nur

teilweise. Solche Verbindungen sind beispielsweise bekannt aus den US-Patenten 4 080 470, 4 215 139, 4 413 010, 4 555 405, 4 608 389 und 4 745 128 sowie den Deutschen Offenlegungsschriften DE-OS 3 320 534 und 3334983.

Es besteht somit noch immer ein Bedürfnis nach Wirkstoffen dieser Substanzklasse mit verbesserten Eigenschaften.

Es wurde nun gefunden, dass die erfindungsgemässen Verbindungen der Formel I bei günstiger Warmblüter-, Fisch- und Pflanzenverträglichkeit wertvolle Wirkstoffe in der Schädlingsbekämpfung sind. Insbesondere betrifft die Anwendung der erfindungsgemässen Wirkstoffe Insekten und Spinnentiere, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwähnten Schädlingen gehören:

aus der Ordnung Lepidoptera zum Beispiel

Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;

aus der Ordnung Coleoptera zum Beispiel

Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp.,Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.; aus der Ordnung der Orthoptera zum Beispiel Blatta spp., Blattella spp., Gryllotalpa spp., Leucophaea maderae, Locusta spp., Periplaneta spp. und Schistocerca spp.;

aus der Ordnung der Isoptera zum Beispiel

Reticulitermes spp.; aus der Ordnung der Psocoptera zum Beispiel Liposcelis spp.; aus der Ordnung der Anoplura zum Beispiel Haematopinus spp., Linognathus spp. Pediculus spp., Pemphigus spp. und Phylloxera spp.; aus der Ordnung der Mallophaga zum Beispiel Damalinea spp. und Trichodectes spp.;

aus der Ordnung der Thysanoptera zum Beispiel

Frankliniella spp., Hercinothrips spp., Taeniothrips spp., Thrips palmi, Thrips tabaci und Scirtothrips aurantii;

aus der Ordnung der Heteroptera zum Beispiel

Cimex spp., Distantiella theobroma, Dysdercus spp., Euchistus spp.,Eurygaster spp., Leptocorisa spp., Nezara spp., Piesma spp., Rhodnius spp., Sahlbergella singularis, Scotinophara spp. und Triatoma spp.;

aus der Ordnung der Homoptera zum Beispiel

Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;

aus der Ordnung der Hymenoptera zum Beispiel

Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;

aus der Ordnung der Diptera zum Beispiel

Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp. Oscinella frit, Pegomyia hyoscyami, Phorbia spp., Rhagoletis

pomonella, Sciara spp., Stomoxys spp., Tabanus spp., Tannia spp. und Tipula spp.;
aus der Ordnung der Siphonaptera z.B.
Ceratophyllus spp., Xenopsylla cheopis,
aus der Ordnung der Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp.; und
aus der Ordnung der Thysanura zum Beispiel
Lepisma saccharina.

Von besonderer Bedeutung hat sich bei den erfindungsgemässen Verbindungen die Verwendung bei der Bekämpfung von Reiszikaden zum Beispiel der Familien Delphacidae und Cicadellidae, wie Nilaparvata lugens, Laodelphax striatellus und Nephotettix cincticeps, erwiesen. Ebenfalls herausragende Wirkung zeigen die Wirkstoffe der Formel I gegen die schwierig unter Kontrolle zu bringenden sogenannten "Weissen Fliegen" der Familie Aleyrodidae mit den Gattungen Bemisia und Trialeurodes, wie Bemisia tabaci oder Trialeurodes vaporarium. Sehr gute Wirkungen erzielen die Verbindungen der Formel I gegen Obstbaumschädlinge der Familien Tortricidae und Olethreutidae, z.B. mit den Gattungen Cydia, Adoxophyes und Lobesia, z.B. mit den Arten Cydia pomonella, Adoxophyes orana und Lobesia botrana. Bei der Bekämpfung von tierparasitären Schädlingen, insbesondere an Haus- und Nutztieren, kommen vor allem Ektoparasiten wie zum Beispiel Milben und Zecken, wie Boophilus microplus und Dermanyssus gallinae, Dipteren wie z.B. Lucilia sericata, und Flöhe wie zum Beispiel Ctenocephalides felis, in Betracht.

Im wesentlichen bewirken die Verbindungen der Formel I in den Zielgruppen von Schädlingen eine Wachstumshemmung oder Entwicklungshemmung bei den verschiedenen Entwicklungsstufen, so dass die Verminderung des Schädlingsbefalls auf Entwicklungsstörungen der Schädlinge, insbesondere einen chemosterilisierenden und oviziden Effekt zurückzuführen ist.

Wegen ihrer vorteilhaften biologischen Wirkung sind unter den Verbindungen der Formel I solche hervorzuheben, worin
$R_1$ für $C_1$-$C_4$-Alkyl, $R_2$ für $C_1$-$C_4$-Alkyl, $C_1$-$C_4$-Alkoxy, $C_1$-$C_4$-Dialkylamino, $C_1$-$C_4$- Alkoxycarbonyl oder -$CONR_{10}R_{11}$ und $R_5$ und $R_6$ beide entweder für Fluor oder für Chlor oder $R_5$ für Chlor oder Fluor und $R_6$ für Wasserstoff, stehen, wobei $R_{10}$ Wasserstoff oder $C_1$-$C_4$-Alkyl, $R_{11}$ Wasserstoff, $C_1$-$C_4$-Alkyl oder Phenyl oder $R_{10}$ und $R_{11}$ zusammen eine $C_4$-$C_6$-Alkylenkette, die durch Sauerstoff unterbrochen sein kann, bedeuten.

Aus dieser Gruppe von bevorzugten Verbindungen sind wiederum solche bevorzugt, worin $R_2$ für $C_1$-$C_4$-Alkyl, $C_1$-$C_4$-Alkoxy, $C_1$-$C_4$-Alkoxycarbonyl, $C_1$-$C_2$-Dialkylamino, Di-($C_1$-$C_4$-alkyl)-aminocarbonyl oder $C_4$-$C_6$-Alkylenaminocarbonyl steht.

Eine besonders bevorzugte Gruppe von Verbindungen der Formel I ist dadurch gekennzeichnet, dass $R_1$ für $C_1$-$C_4$-Alkyl, $R_2$ für $C_1$-$C_4$-Alkyl, $C_1$-$C_4$-Alkoxy, Di-($C_1$-$C_2$-alkyl)-aminocarbonyl, oder $C_1$-$C_4$-Alkoxycarbonyl, $R_3$ für Methyl oder Wasserstoff, $R_4$ für Wasserstoff und $R_5$ und $R_6$ für Fluor oder Chlor stehen.

Bevorzugte Einzelverbindungen gemäss vorliegender Erfindung sind:

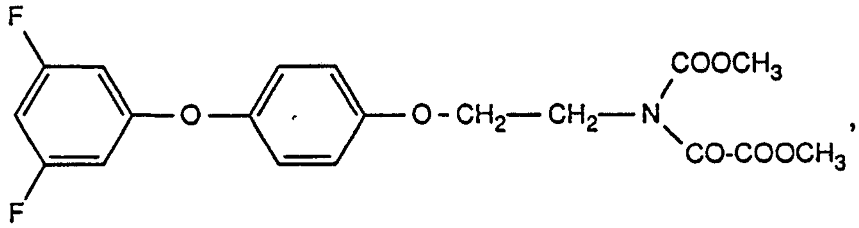

,

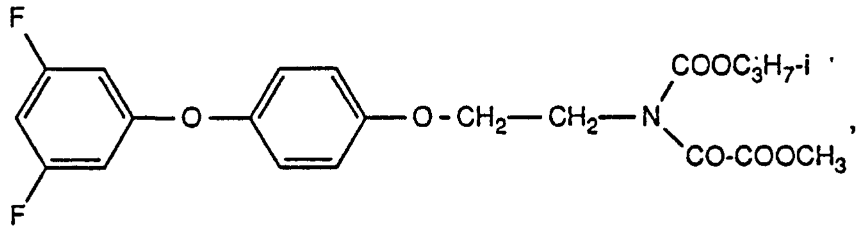

,

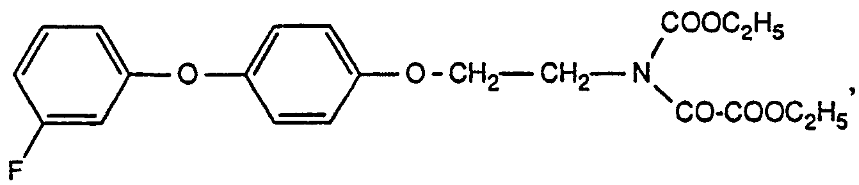

,

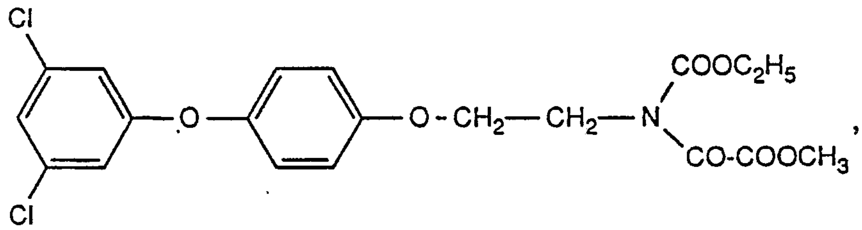,

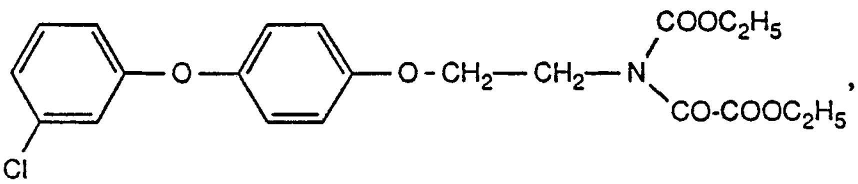,

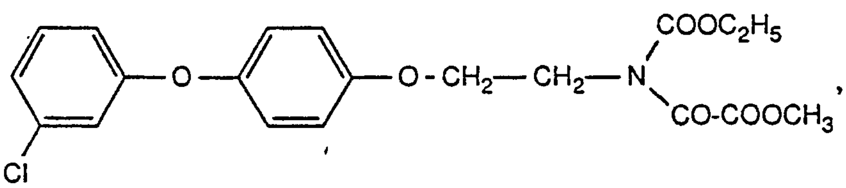,

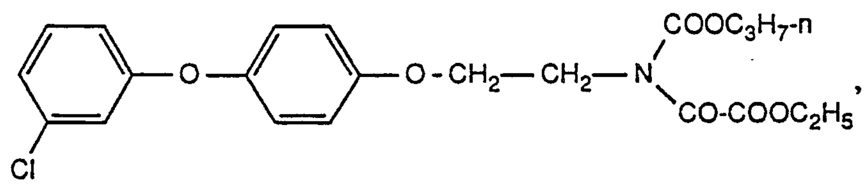,

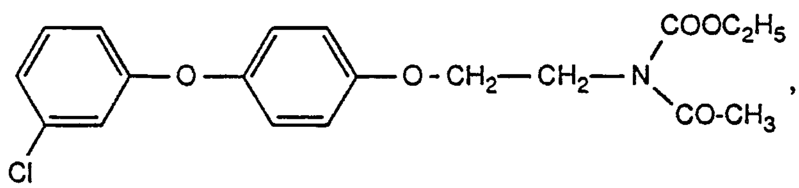,

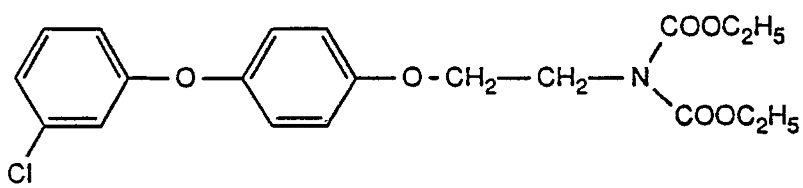,

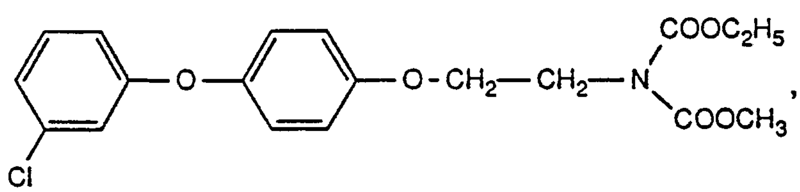,

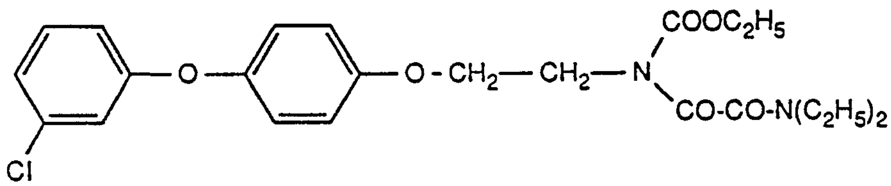

,

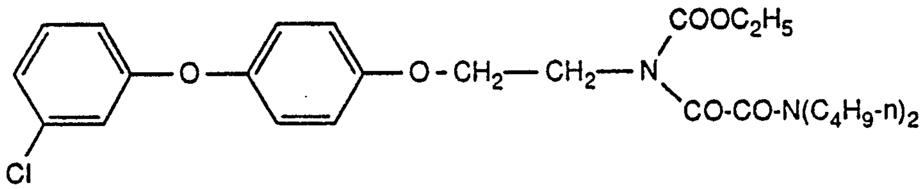

,

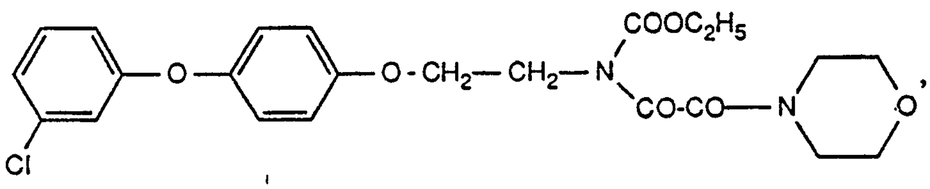

,

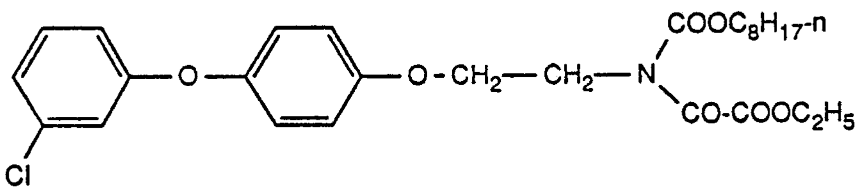

,

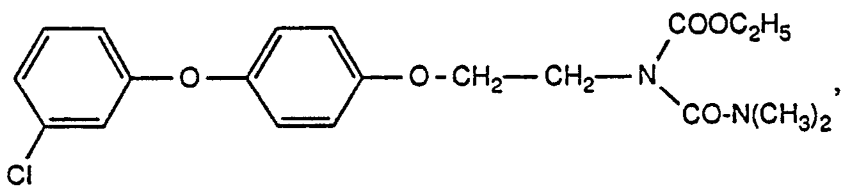

,

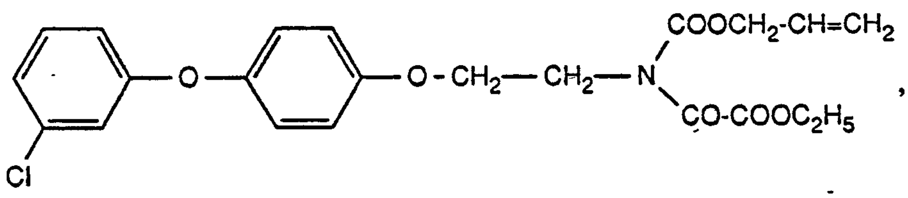

,

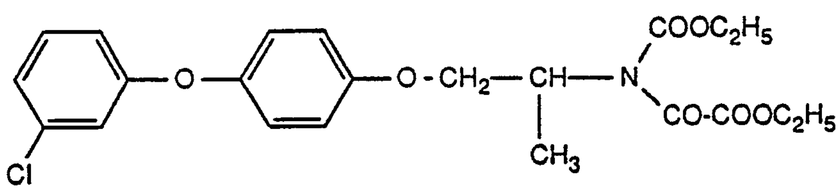

,

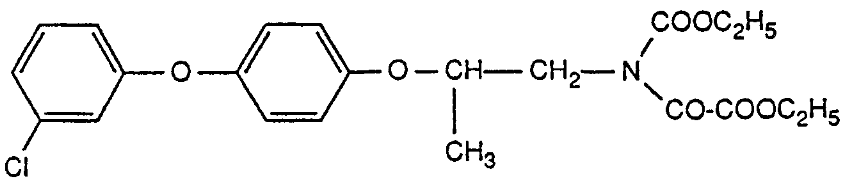

,

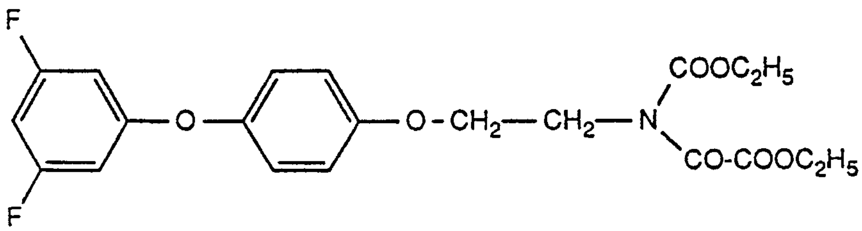

und

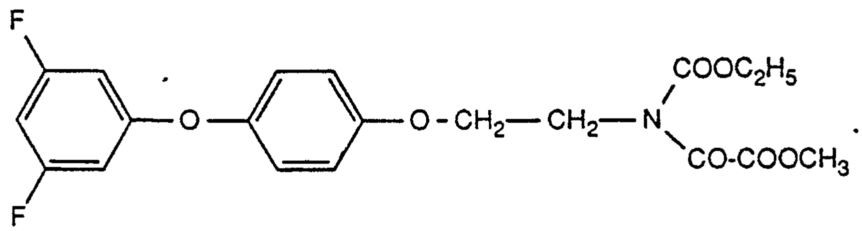

.

Die erfindungsgemässen Verbindungen der Formel I können nach an sich bekannten Methoden hergestellt werden. So kann man die Verbindungen der Formel I beispielsweise erhalten, indem man einen Halogenphenoxyphenoxyäthylcarbaminsäureester der Formel II

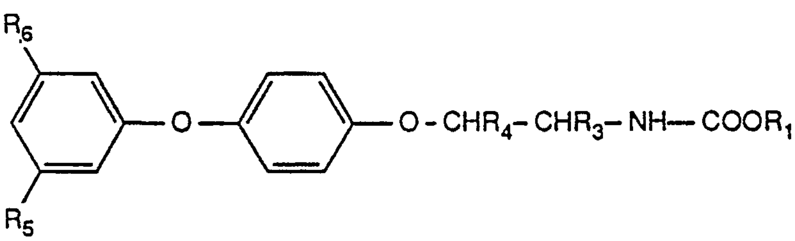

(II)

worin $R_1$, $R_3$, $R_4$, $R_5$ und $R_6$ die unter Formel I angegebenen Bedeutungen haben, mit einer Acylverbindung der Formel III

$X\text{-}CO\text{-}R_2$ (III)

worin $R_2$ die unter Formel I angegebene Bedeutung hat und X für Chlor, Brom, $-O\text{-}CO\text{-}R_2$ oder $-O\text{-}CO(C_1\text{-}C_4\text{-Alkyl})$ steht, acyliert.

Das erfindungsgemässe Herstellungsverfahren wird vorzugsweise in Gegenwart einer Base durchgeführt. Als Basen eignen sich sowohl anorganische Basen, wie Alkali- und Erdalkalicarbonate oder -hydrogencarbonate, beispielsweise Natriumcarbonat, Kaliumcarbonat, Natriumhydrogencarbonat oder Kaliumhydrogencarbonat, oder Alkali- und Erdalkalihydride, wie Kaliumhydrid, Natriumhydrid oder Calciumhydrid, als auch organische Basen, wie tertiäre Amine, beispielsweise Trialkylamine wie Triäthylamin, Diisopropyläthylamin, Pyridin, Dimethylaminopyridin, 1,4-Diazabicyclo-[2,2,2]octan oder 1,8-Diazabicyclo-[5,4,0]undec-7-en; Alkalialkoholate, wie Natriummethylat, Natriumäthylat oder Kalium-tert.butylat oder Alkalialkylverbindungen, wie Butyllithium.

Mit Vorteil werden die Umsetzungen zur Herstellung der erfindungsgemässen Verbindungen der Formel I in inerten, aprotischen organischen Lösungsmitteln durchgeführt. Solche Lösungsmittel sind Kohlenwasserstoffe, wie Hexan, Heptan, Cyclohexan, Ligroin, Benzol, Toluol, Xylol, Mesitylen oder Tetralin; chlorierte Kohlenwasserstoffe, wie Dichlormethan, Chloroform, Tetrachlorkohlenstoff, Chlorbenzol, Dichlorbenzol, Trichloräthan oder Tetrachloräthan; Aether, wie Diäthyläther, 1,2-Dimethoxyäthan, Tetrahydrofuran oder Dioxan; Nitrile, wie Acetonitril oder Propionitril; Dimethylsulfoxid oder Sulfolan; oder Dialkylformamide, wie

Dimethylformamid oder Dimethylacetamid.

Die Reaktionstemperaturen des erfindungsgemässen Verfahrens liegen je nach Wahl des Lösungsmittels und der fakultativ zu verwendenden Base im allgemeinen zwischen -10° C und dem Siedepunkt des Reaktionsgemisches, meist zwischen 0° und +130° C. Bevorzugte Reaktionstemperaturen liegen zwischen +20° C und +100° C. Bei Verwendung sehr reaktiver Reagenzien, wie beispielsweise Butyllithium, wird die Reaktionstemperatur vorzugsweise beträchtlich tiefer, etwa zwischen -80° C und +20° C, gehalten. Bevorzugt ist hier zum Beispiel der Bereich zwischen -70° C und 0° C.

In einer Variante des obigen Verfahrens kann man die Verbindungen der Formel I, worin $R_2$ für die Gruppe $-CO-R_7$ steht auch erhalten, indem man den Carbaminsäureester der Formel II zunächst mit Oxalsäuredichlorid umsetzt und das erhaltene Zwischenprodukt der Formel

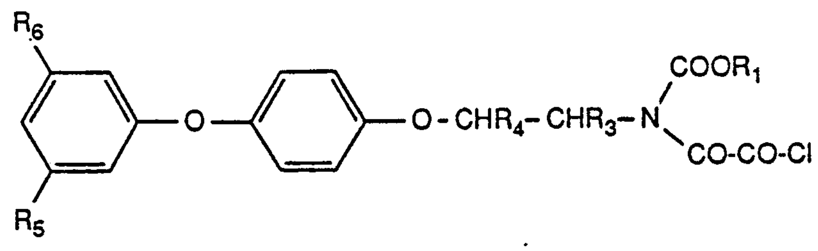

worin $R_1$, $R_3$, $R_4$, $R_5$ und $R_6$ die unter Formel I gegebenen Bedeutungen haben, in Gegenwart einer Base mit einem Alkohol oder einem Amin der Formel
$H-R_7$
worin $R_7$ die unter Formel I gegebene Bedeutung hat, umsetzt.

Die Ausgangsmaterialien der Formeln II und III sind zum grossen Teil bekannt. Neue Einzelverbindungen, welche unter die Formeln II und III fallen, können nach bekannten Methoden hergestellt werden. So erhält man die Ausgangsprodukte der Formel II auf einfachem Wege durch Umsetzung eines 2-[4-(Halogenphenoxy)-phenoxy]-äthylamins der Formel IV

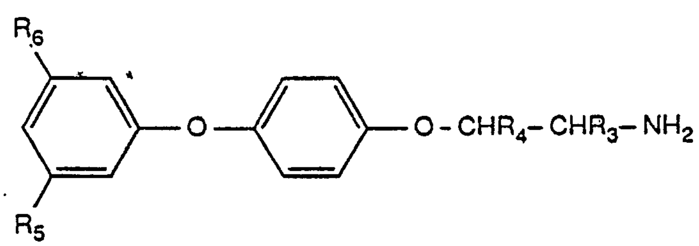

(IV)

worin $R_3$, $R_4$, $R_5$ und $R_6$ die unter Formel I gegebenen Bedeutungen haben, mit einem Halogenameisensäureester der Formel V
$Hal-CO-OR_1$ (V),
worin $R_1$ die unter Formel I gegebene Bedeutung hat, und Hal für Halogen, vorzugsweise Brom oder Chlor steht, in Gegenwart einer Base. Die Verbindungen der Formel II können ferner hergestellt werden durch Umsetzung eines Phenoxyphenols der Formel

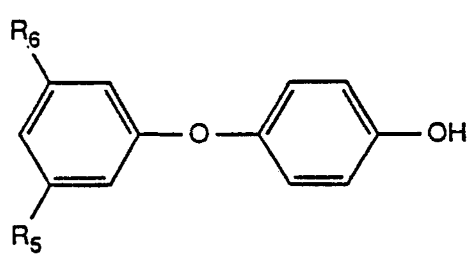

mit einem 2-Halogenäthylcarbaminsäureester der Formel

Hal-CH($R_4$)-CH($R_3$)-NH-CO-O$R_1$
in Gegenwart einer Base, wobei $R_1$, $R_3$, $R_4$, $R_5$ und $R_6$ die oben angegebenen Bedeutungen haben und Hal für ein Halogenatom, vorzugsweise Chlor oder Brom, steht.

Die Verbindungen der Formel IV und V sind bis auf die 1-Methyläthylamine der Formel IVa

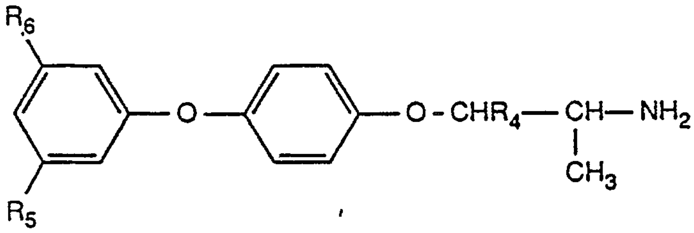

(IVa)

worin $R_4$, $R_5$ und $R_6$ die unter Formel I gegebenen Bedeutungen haben, bekannt. Diese neuen Zwischenprodukte der Formel IVa können auf dem folgenden Wege durch reduktive Aminierung hergestellt werden:

Schema 1

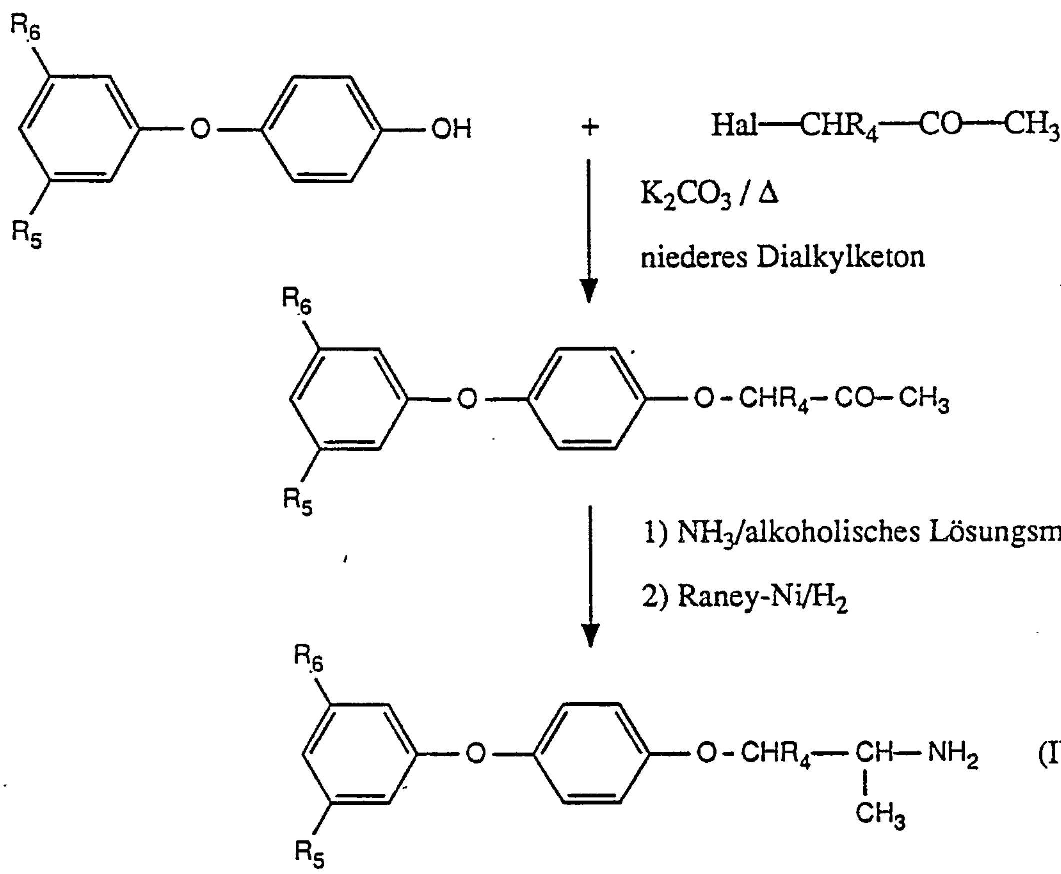


(IVa)

$R_4$, $R_5$ und $R_6$ haben die unter Formel I gegebenen Bedeutungen; Hal bedeutet Chlor oder Brom.

Die Verbindungen IVa sind neu und bilden zusammen mit dem Verfahren zu ihrer Herstellung ebenfalls einen Gegenstand vorliegender Erfindung.

Die Wirkung der erfindungsgemässen Verbindungen, beziehungsweise der sie enthaltenden Mittel, lässt sich durch Zusatz von anderen Insektiziden und/oder Akariziden wesentlich verbreitern und an gegebene Umstände anpassen. Als solche Zusätze kommen zum Beispiel organische Phosphorverbindungen, Nitrophenole und deren Derivate, Formamidine, Harnstoffe, Carbamate, Pyrethroide, chlorierte Kohlenwasserstoffe und Bacillus thuringiensis-Präparate in Betracht.

Mit besonderem Vorteil kann man die Verbindungen der Formel I auch mit Substanzen kombinieren, welche einen pestizid verstärkenden Effekt ausüben. Beispiele solcher Ver bindungen sind unter anderen: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die Verbindungen der Formel I werden in unveränderter Form oder vorzugsweise zusammen mit den in der Formulierungstechnik üblichen Hilfsmitteln eingesetzt und werden daher beispielsweise zu Emulsionskonzentraten, direkt versprühbaren oder verdünnbaren Lösungen, verdünnten Emulsionen, Spritzpulvern, löslichen Pulvern, Stäubemitteln, Granulaten oder auch Verkapselungen in zum Beispiel polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, das heisst die den Wirkstoff der Formel I oder Kombinationen dieser Wirkstoffe mit andern Insektiziden oder Akariziden, und gegebenenfalls einen festen oder flüssigen Zusatzstoff enthaltenden Mittel, Zubereitungen oder Zusammensetzungen, werden in bekannter Weise hergestellt, wie durch inniges Vermischen und/oder Vermahlen der Wirkstoffe mit Streckmitteln, wie zum Beispiel mit Lösungmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$-$C_{12}$, wie zum Beispiel Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethlenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle, wie epoxydiertes Kokosnussöl oder Sojaöl, oder Wasser.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden.

Als gekörnte, adsorptive Granulatträger kommen sowohl poröse Typen, wie zum Beispiel Bimsstein, Ziegelbruch, Sepiolit oder Bentonit, als auch nicht sorptive Trägermaterialien wie Calcit oder Sand in Frage. Darüberhinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach der Art des zu formulierenden Wirkstoffes der Formel I oder der Kombinationen dieser Wirkstoffe und andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen wie wasserlöslich synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, zum Beispiel das Natrium- oder Calcium-Salz der Ligninsulfonsäure, des Dodecylschwefelsäureesters oder eines aus natürlichen Fettsäuren hergestellten Fettalkoholsulfatgemisches. Hierher gehären auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8 - 22 C-Atomen. Arylalkylsulfonate sind beispielsweise die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie die Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddükte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Proplyenglykoleinheit 1 bis 5 Aethylenglykoleinheiten.

Als Beispiele nichtionischer Tenside seien Nonylphenolpolyäthoxyäthanole, Ricinusölpolyglykoläther, Polypropylen-Poly äthylenoxidaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, zum Beispiel das Stearyltrimethylammoniumchlorid oder das Benzyl-di-(2-chloräthyl)-äthylammoniumbromid.

Die in der Formulierungstechnik gebräuchlichen Tenside sind unter anderem in den folgenden Publikationen beschrieben:

"1985 International Mc Cutcheon's Emulsifiers & Detergents", Glen Rock NJ USA, 1985",
H. Stache, "Tensid-Taschenbuch", 2. Aufl., C. Hanser Verlag München, Wien 1981,
M. and J. Ash. "Encyclopedia of Surfactants", Vol. I-III, Chemical Publishing Co., New York, 1980-1981.

Die pestiziden Zubereitungen enthalten in der Regel 0,1 bis 99 %, insbesondere 0,1 bis 95 %, des Wirkstoffs der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 10 bis 1000 g Wirkstoff pro Hektar, vorzugsweise 25 bis 250 g/ha.

Insbesondere setzen sich bevorzugte Formulierungen folgendermassen zusammen:
(% = Gewichtsprozent)

Emulgierbare Konzentrate
Aktiver Wirkstoff: 1 bis 50 %, bevorzugt 5 bis 30 %
oberflächenaktives Mittel: 5 bis 30 %, vorzugsweise 10 bis 20 %
flüssiges Trägermittel: 20 bis 94 %, vorzugsweise 50 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %

Suspensions-Konzentrate
Aktiver Wirkstoff: 5 bis 75 %, vorzugsweise 10 bis 50 %
Wasser: 94 bis 24 %, vorzugsweise 88 bis 30 %
oberflächenaktives Mittel: 1 bis 40 %, vorzugsweise 2 bis 30 %

Benetzbare Pulver
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermaterial: 5 bis 95 %, vorzugsweise 15 bis 90 %

Granulate
aktiver Wirkstoff: 0,5 bis 30 %, vorzugsweise 3 bis 15 %
festes Trägermittel: 99,5 bis 70 %, vorzugsweise 97 bis 85 %

Die Mittel können auch weitere Zusätze wie Stabilisatoren, Entschäumer, Konservierungsmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Beispiel H1: N-Aethoxyoxalyl-2-[4-(3,5-difluorphenoxy)-phenoxy]-äthylcarbaminsäure-äthylester

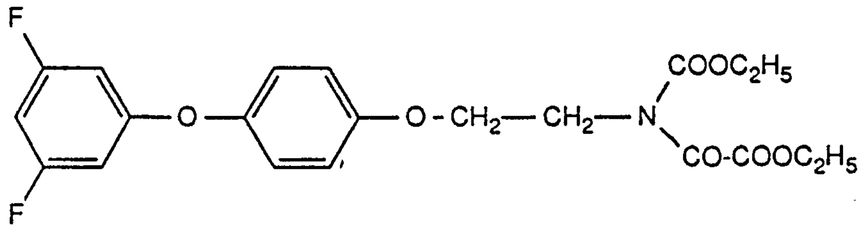

Zu der Lösung von 9,2 g 2-[4-(3,5-Difluorphenoxy)-phenoxy]-äthylcarbaminsäure-äthylester und 0,2 g 4-Dimethylaminopyridin in 70 ml 1,2-Dichloräthan lässt man unter Rühren und unter einer Stickstoffatmosphäre bei +80° C innerhalb von 15 Minuten die Lösung von 8 g Oxalsäure-monoäthylester-chlorid in 10 ml 1,2-Dichloräthan zutropfen. Anschliessend rührt man das Gemisch für 15 Stunden bei +80° C. Nach dem Abkühlen auf Raumtemperatur wird das Reaktionsgemisch mit Wasser, 0,1N-Salzsäure und schliesslich wieder mit Wasser gewaschen und über Natriumsulfat getrocknet. Das Lösungsmittel wird im Vakuum vollständig abdestilliert. Der so erhaltene N-Aethoxyoxalyl-N-2-[4-(3,5-difluorphenoxy)-phenoxy]-äthylcarbaminsäure-äthylester wird an Kieselgel chromatographisch gereinigt (Eluierungsmittel: n-Hexan/Diäthyläther, 5:1) und schliesslich aus n-Hexan unter Zusatz von wenig Aether umkristallisiert. Man erhält farblose Kristalle der Titelverbindung vom Smp. 73-75° C.

Beispiel H2: N-Methoxyoxalyl-2-[4-(3,5-difluorphenoxy)-phenoxy]-äthylcarbaminsäure-äthylester

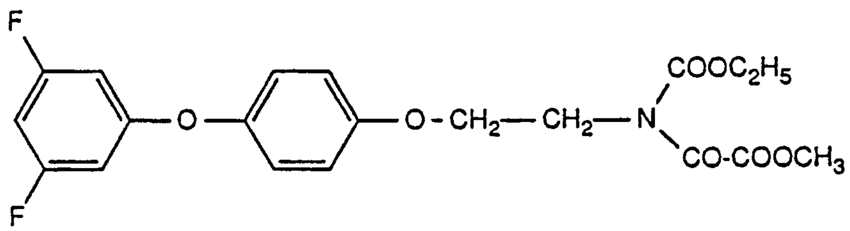

Zu der Lösung von 9,2 g 2-[4-(3,5-Difluorphenoxy)-phenoxy]-äthylcarbaminsäure-äthylester und 0,2 g 4-Dimethylaminopyridin in 70 ml 1,2-Dichloräthan lässt man unter Rühren und unter einer Stickstoffatmosphäre bei +80° C innerhalb von 15 Minuten die Lösung von 8 g Oxalsäure-monomethylesterchlorid in 10 ml 1,2-Dichloräthan zutropfen. Anschliessend rührt man für 15 Stunden bei +80° C. Nach dem Abkühlen auf Raumtemperatur wird das Reaktionsgemisch mit Wasser, 0,1N-Salzsäure und schliesslich wieder mit Wasser neutral gewaschen und über Natriumsulfat getrocknet. Das Lösungsmittel wird im Vakuum vollständig abdestilliert. Der so erhaltene N-Methoxyoxalyl-2-[4-(3,5-difluorphenoxy)-phenoxy]-äthylcarbaminsäureäthylester wird an Kieselgel chromatographisch gereinigt (Eluierungsmittel: n-Hexan/Diäthyläther, 5:1) und schliesslich aus n-Hexan unter Zusatz von wenig Aether umkristallisiert: farblose Kristalle vom Smp. 56-58° C.

Beispiel H3: N-Aethoxyoxalyl-2-[4-(3,5-difluorphenoxy)-phenoxy]-äthylcarbaminsäuremethylester

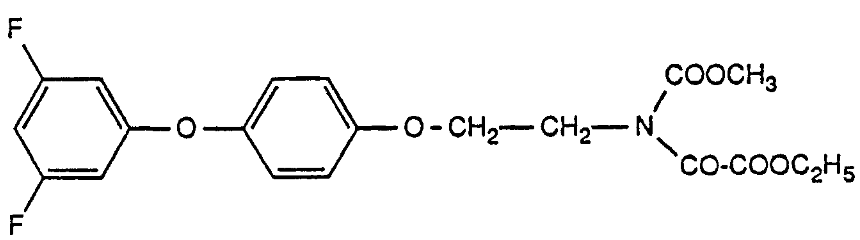

a) Zu einer Lösung von 17,1 g 4-(3,5-Difluorphenoxy)-phenol in 100 ml Dimethylformamid gibt man 21,3 g Kaliumcarbonat-Pulver, 1,5 g fein pulverisiertes Kaliumjodid und 16 g 2-Chloräthylcarbaminsäure-methylester zu und erhitzt das Reaktionsgemisch für 15 Stunden auf +95 °C. Hierauf wird das Reaktionsgemisch abgekühlt, auf Eiswasser gegossen und wiederholt mit Aether extrahiert. Die vereinigten Aetherphasen werden mit Wasser gewaschen und über Natriumsulfat getrocknet. Das Lösungsmittel wird voll ständig abdestilliert. Das Rohprodukt wird durch Chromatographie an Kieselgel weiter gereinigt (Eluierungsmittel:Petroläther (40-65 °C)/Diäthyläther, 5:1), wodurch der 2-[4-(3,5-Difluorphenoxy)-penoxy]-äthylcarbaminsäure-methylester erhalten wird, $n^{21}_D$: 1.5394.

Analog werden aus den entsprechenden 2-Chloräthylcarbaminsäure-äthyl-, isopropyl-, -n-propyl- und -allylestern und den 3,5-Difluor- oder 3-Chlorphenoxy-phenolen die folgenden Carbaminsäure-alkylester hergestellt:

2-[4-(3,5-Difluorphenoxy)-phenoxy]-äthylcarbaminsäure-isopropylester, $n^{21}_D$: 1.5243;
2-[4-(3-Chlorphenoxy)-phenoxy]-äthylcarbaminsäure-methylester, $n^{20}_D$: 1.5719;
2-[4-(3-Chlorphenoxy)-phenoxy]-äthylcarbaminsäure-äthylester, Smp.: 45-46 °C;
2-[4-(3-Chlorphenoxy)-phenoxy]-äthylcarbaminsäure-n-propylester, Smp.: 62-63 °C;
2-[4-(3-Chlorphenoxy)-phenoxy]-äthylcarbaminsäure-isopropylester, Smp.: 61-62 °C und
2-[4-(3-Chlorphenoxy)-phenoxy]-äthylcarbaminsäure-n-allylester, Smp.: 51-52 °C.

b) Aus den gemäss a) erhaltenen Zwischenprodukten erhält man in einer Arbeitsweise wie im Beispiel H1 unter Verwendung von Oxalsäure-monomethylester-chlorid oder Oxalsäure-monoäthylester-chlorid die folgenden erfindungsgemässen Endprodukte:

N-Aethoxyoxalyl-2-[4-(3,5-difluorphenoxy)-phenoxy]-äthylcarbaminsäure-methylester, $n^{20}_D$: 1.5230;
N-Methoxyoxalyl-2-[4-(3,5-difluorphenoxy)-phenoxy]-äthylcarbaminsäure-isopropylester, $n^{21}_D$: 1.5152;
N-Aethoxyoxalyl-2-[4-(3-chlorphenoxy)-phenoxy]-äthylcarbaminsäure-äthylester, $n^{20}_D$: 1.5396 und
N-Aethoxyoxalyl-2-[4-(3-chlorphenoxy)-phenoxy]-äthylcarbaminsäure-allylester, $n^{20}_D$: 1.5441.

<u>Beispiel H4</u>: <u>N-Methoxyoxalyl-2-[4-(3-chlorphenoxy)-phenoxyl]-1-methyläthylcarbaminsäure-äthylester</u>

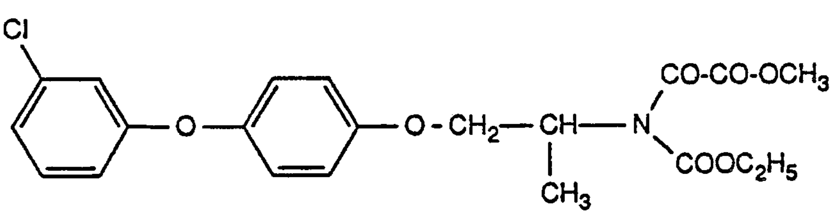

a) Zu der Lösung von 39,8 g 4-(3-Chlorphenoxy)-phenol in 250 ml Aethylmethylketon setzt man 32 g pulverisiertes Kaliumcarbonat sowie 2 g feinpulveriges Kaliumjodid zu und erwärmt auf Rückflusstemperatur. Unter Rühren lässt man innerhalb einer Stunde 25 g frisch destilliertes Chloraceton zutropfen und rührt für weitere 2 Stunden bei Rückflusstemperatur. Nach dem Abkühlen wird das Reaktionsgemisch filtriert, das Lösungsmittel im Vakuum abdestilliert und das Rohprodukt durch Kieselgel filtriert, wodurch das reine, farblose 1-[4-(3-Chlorphenoxy)-phenoxy]-2-propanon erhalten wird, $n^{20}_D$: 1.5788.

b) 51 g 1-[4-(3-Chlorphenoxy)-phenoxy]-2-propanon werden in einem Autoklaven in 510 ml Methanol gelöst und mit 10 g Raney-Nickel versetzt. Es werden 31 g flüssiges Ammoniak eingefüllt und dann wird Wasserstoffgas aufgepresst. Das Reaktionsgemisch wird bei 50 bar und +40-45 °C für 2 Stunden hydriert. Das Reaktionsgemisch wird nun durch Diatomeenerde filtriert und das Lösungsmittel im Vakuum vollständig abdestilliert. Durch Chromatographie des Rohproduktes an Kieselgel (Eluierungsmittel: Diäthyläther/Methanol, 9:1) wird das reine 2-Amino- 1-[4-(3-chlorphenoxy)-phenoxy]-propan als farblose, ölartige Flüssigkeit erhalten, $n^{20}_D$: 1.5743.

c) Zu der Lösung von 31,5 g 2-Amino-1-[4-(4-fluorphenoxy)-phenoxy]-propan, 20 g Diisopropyläthylamin und 1,0 g 4-Dimethylaminopyridin in 120 ml Toluol lässt man unter Rühren bei 20-22 °C innerhalb von 30 Minuten die Lösung von 13,5 g Chlorameisensäureäthylester in 20 ml Toluol zutropfen. Anschliessend wird für 15 Stunden bei Raumtemperatur gerührt Zur Aufarbeitung wird das Reaktionsgemisch auf 500 ml Eiswasser gegossen und dreimal mit Aether extrahiert. Die vereinigten organischen Phasen werden zweimal mit kalter 1N-Salzsäure und mit Wasser gewaschen. Nach dem Trocknen der organischen Lösung über Natriumsulfat wird das Lösungsmittel abdestilliert und das Rohprodukt durch Chromatographie an Kieselgel

gereinigt (Eluierungsmittel: n-Hexan/Diäthyläther, 5:1), wodurch der reine 2-[4-(3-Chlorphenoxy)-phenoxy]-1-methyläthylcarbaminsäure-äthylester als blassgelbe, ölartige Flüssigkeit erhalten wird; $n^{21}_D$: 1.5530.

d) Zu der Lösung von 9,0 g 2-[4-(3-Chlorphenoxy)-phenoxy]-1-methyläthylcarbaminsäure-äthylester und 0,2 g 4-Dimethylaminopyridin in 50 ml 1,2-Dichloräthan lässt man unter Rühren und unter einer Stickstoffatmosphäre bei +80° C innerhalb von 20 Minuten die Lösung von 6,3 g Oxalsäure-monomethylester-chlorid in 10 ml 1,2-Dichloräthan eintropfen. Anschliessend wird das Gemisch für 17 Stunden bei +80° C gerührt. Nach dem Abkühlen auf Raumtemperatur wird das Reaktionsgemisch nacheinander mit gekühlter gesättigter Natriumhydrogencarbonat-Lösung, mit 0,1N Salzsäure und schliesslich mit Wasser gewaschen und über Natriumsulfat getrocknet. Das 1,2-Dichlormethan wird im Vakuum vollständig abdestilliert. Der so erhaltene N-Methoxyoxalyl-2-[4-(3-chlorphenoxy)-phenoxy]-1-methyläthylcarbaminsäure-äthylester kann, falls erwünscht, an Kieselgel chromatographiert werden (Eluierungsmittel: n-Hexan/Dichlormethan, 3:1); $n^{20}_D$: 1.5443.

Beispiel H5: N-(Aethoxyoxalyl)-2-[4-(3-chlorphenoxy)-phenoxy]-propylcarbaminsäure-äthylester

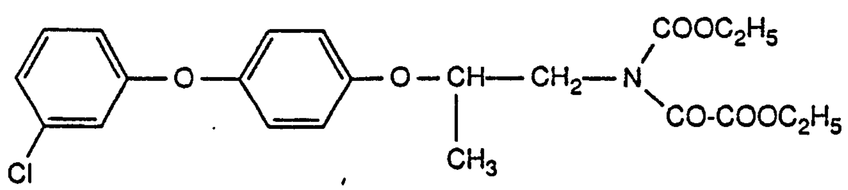

a) Zu einer Lösung von 25,2 g 3-Chlorphenoxy-phenol in 40 ml Dimethylsulfoxid gibt man unter Rühren und leichter Aussenkühlung die Lösung von 15 g Kalium-tert.butylat in 45 ml Dimethylsulfoxid zu, fügt 1,5 g 18-Crown-6 zu und lässt nun bei 15° C innerhalb 1 Stunde die Lösung von 28,3 g 2-Methylsulfonyloxy-propylcarbaminsäure-äthylester in 30 ml Dimethylsulfoxid zutropfen und rührt erst 5 Stunden bei 20-22° C und anschliessend 19 Stunden bei +55° C. Nach dem Abkühlen wird das Reaktionsgemisch auf Eiswasser gegossen, wiederholt mit Aether extrahiert, die vereinigten Aetherphasen mit Wasser gewaschen und das Lösungsmittel abdestilliert. Das Rohprodukt wird durch Chromatographie an Kieselgel weiter gereinigt (Eluierungsmittel: Diäthyläther/Hexan, 1:9), wodurch der 2-[4-(3-Chlorphenoxy)-phenoxy]-propylcarbaminsäure-äthylester als blassgelbes, viskoses Oel erhalten wird, $n^{20}_D$: 1.5500.

b) Zu einer Lösung von 5,5 g 2-[4-(3-Chlorphenoxy)-phenoxy]-propylcarbaminsäure-äthylester und 0,2 g 4-Dimethylaminopyridin in 50 ml 1,2-Dichloräthan lässt man unter Rühren bei +80° C in 10 Minuten 4,3 g Oxalsäure-monoäthylester-chlorid zutropfen. Hierauf rührt man das Gemisch für weitere 25 Stunden bei +80° C. Nach dem Abkühlen auf Raumtemperatur wird das Reaktionsgemisch wiederholt mit Wasser und gesättigter Natriumhydrogencarbonat-Lösung gewaschen und über Natriumsulfat getrocknet. Das Lösungsmittel wird im Vakuum vollständig abdestilliert. Der als Rohprodukt erhaltene N-(Aethoxyoxalyl)-2-[4-(3-chlorophenoxy)-phenoxy]-propylcarbaminsäure-äthylester wird an Kieselgel chromatographisch gereinigt (Eluierungsmittel: Diäthyläther/n-Hexan, 1:6) und so in reiner Form als viskose, ölartige Flüssigkeit erhalten $n^{20}_D$: 1.5303.

Beispiel H6: N-Aethoxycarbonyl-2-[4-(3-chlorphenoxy)-phenoxyl-äthylcarbaminsäure-äthylester

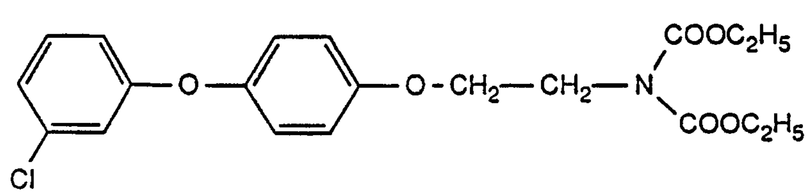

1,50 g einer 55%-igen Natriumhydrid-Dispersion in Mineralöl werden wiederholt mit n-Hexan gewaschen und in 30 ml Tetrahydrofuran suspendiert. Zu dieser Suspension lässt man bei Raumtemperatur unter Rühren die Lösung von 11,5 g 2-[4-(3-Chlorphenoxy)-phenoxy]-äthylcarbaminsäure-äthylester in 30 ml Tetrahydrofuran zutropfen und rührt weitere 5 Stunden bei Raumtemperatur bis das Natriumhydrid

vollständig umgesetzt ist. Hierauf lässt man bei 0-5°C innerhalb von 10 Minuten die Lösung von 4,6 g frisch destilliertem Chlorameisensäureäthylester in 10 ml Tetrahydrofuran zutropfen und rührt für weitere 50 Minuten bei Raumtemperatur. Nun wird das Reaktionsgemisch auf Eiswasser gegossen und wiederholt mit Aether extrahiert. Die vereinigten Aetherphasen wäscht man zweimal mit kalter, gesättigter Natriumhydrogencarbonatlösung, anschliessend mit Wasser und Natriumchloridlösung, trocknet die organische Phase über Natriumsulfat und destilliert das Lösungsmittel ab. Das Rohprodukt wird an Kieselgel chromatographisch weiter gereinigt (Eluierungsmittel: n-Hexan/Diäthyläther, 5:1) wodurch der N-Aethoxycarbonyl-2-[4-(3-chlorphenoxy)-phenoxy]-äthylcarbaminsäure-äthylester als farbloses, viskoses Oel erhalten wird, $n^{24}_D$: 1.5426.

Analog werden hergestellt:

Aus 2-[4-(3-Chlorphenoxy)-phenoxy]-äthylcarbaminsäure-äthylester und

a) Acetylchlorid der N-Acetyl-2-[4-(3-chlorphenoxy)-phenoxy]-äthylcarbaminsäure-äthylester, $n^{20}_D$: 1.5542;

b) Chlorameisensäuremethylester der N-Methoxycarbonyl-2-[4-(3-chlorphenoxy)-phenoxy]-äthylcarbaminsäure-äthylester, $n^{20}_D$: 1.5493;

c) n-Buttersäurechlorid der N-(n-Butyroyl)-2-[4-(3-chlorphenoxy)-phenoxy]-äthylcarbaminsäure-äthylester, $n^{20}_D$: 1.5469 und

d) Dimethylcarbamoylchlorid der N-(Dimethylcarbamoyl)-2-[4-(chlorphenoxy)-phenoxy]-äthylcarbaminsäure-äthylester, $n^{21}_D$: 1.5490.

Beispiel H7: N-Diäthylaminooxalyl-2-[4-(3-chlorphenoxy)-phenoxy]-äthylcarbaminsäure-äthylester

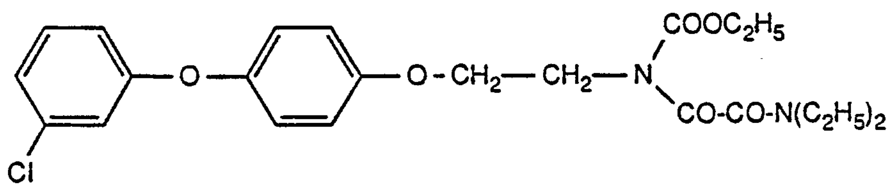

a) Zu einer Lösung von 33,6 g 2-[4-(3-Chlorphenoxy)-phenoxy]-äthylcarbaminsäure-äthylester in 100 ml 1,2-Dichloräthan lässt man bei Raumtemperatur unter Rühren 25,4 g Oxalylchlorid zutropfen, erhitzt auf Rückflusstemperatur und rührt weitere 4 Stunden bei Siedetemperatur bis kein Chlorwasserstoffgas mehr entwickelt wird. Nun wird unter einer Stickstoffatmosphäre das Lösungsmittel und das überschüssige Oxalylchlorid abdestilliert und der so erhaltene N-Chloroxalyl-2-[4-(3-chlorphenoxy)-phenoxy]-äthylcarbaminsäure-äthylester im Hochvakuum vollständig entgast, $n^{21}_D$: 1.5521.

b) Zu einer Lösung von 8,5 g N-Chloroxalyl-2-[4-(3-chlorphenoxy)-phenoxy]-äthylcarbaminsäure-äthylester in 80 ml Toluol lässt man unter Rühren bei 0-5°C innerhalb von 30 Minuten die Lösung von 4,4 g Diäthylamin in 20 ml Toluol zutropfen. Anschliessend wird für 2 Stunden bei Raumtemperatur gerührt. Nun wird das Reaktionsgemisch nacheinander wiederholt mit eiskalter 1N-Salzsäure, mit 10%-iger Natriumhydrogencarbonatlösung und mit Wasser gewaschen und über Natriumsulfat getrocknet. Das Lösungsmittel wird vollständig abdestilliert. Durch Chromatographie an Kieselgel (Eluierungsmittel: n-Hexan/Diäthyläther, 6:1) wird der reine N-(Diäthylamino-oxalyl)-2-[4-(3-chlorphenoxy)-phenoxy]-äthylcarbaminsäure-äthylester erhalten, $n^{20}_D$: 1.5471.

Analog zur Arbeitsweise b) werden aus dem N-Chloroxalyl-2-[4-(3-chlorphenoxy)-phenoxy]-äthylcarbaminsäure-äthylester und Morpholin oder Dibutylamin die folgenden Verbindungen hergestellt:

N-(N-Morpholinooxalyl)-2-[4-(3-chlorphenoxy)-phenoxy]-äthylcarbaminsäure-äthylester, $n^{21}_D$: 1.5569 und

N-Dibutylaminooxalyl-2-[4-(3-chlorphenoxy)-phenoxy]-äthylcarbaminsäure-äthylester, $n^{20}_D$: 1.5345.

In analoger Weise kann man die folgenden Wirkstoffe der Formel I erhalten:

Tabelle 1:

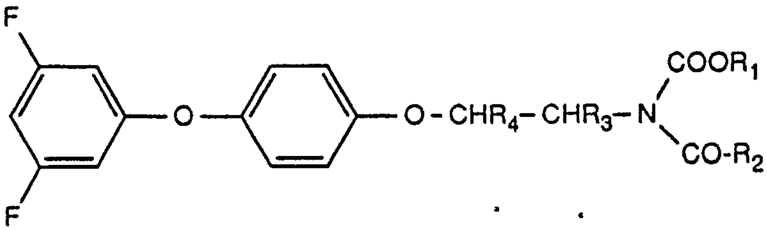

| Verb. Nr. | $R_1$ | $R_2$ | $R_3$ | $R_4$ | Physikalische Daten |
|---|---|---|---|---|---|
| 1.01 | $C_2H_5$ | $COOC_2H_5$ | H | H | Smp. 73-75°C |
| 1.02 | $C_2H_5$ | $COOC_4H_9$-t | H | H | |
| 1.03 | $C_2H_5$ | $COOCH_3$ | H | H | Smp. 56-58°C |
| 1.04 | $C_2H_5$ | $COOC_2H_5$ | $CH_3$ | H | |
| 1.05 | $CH_3$ | $COOCH_3$ | H | H | |
| 1.06 | $C_3H_7$-n | $COOC_2H_5$ | H | H | |
| 1.07 | $C_4H_9$-n | $COOCH_3$ | H | H | |
| 1.08 | $C_2H_5$ | $COOCH_3$ | $CH_3$ | H | |
| 1.09 | $C_2H_5$ | $COOCH_3$ | $CH_3$ | $CH_3$ | |
| 1.10 | $C_2H_5$ | $CH_3$ | H | H | |
| 1.11 | $C_2H_5$ | $C_3H_7$-n | H | H | |
| 1.12 | $C_2H_5$ | $OC_2H_5$ | H | H | |
| 1.13 | $C_2H_5$ | $OCH_3$ | H | H | |
| 1.14 | $C_2H_5$ | $OC_4H_9$-n | H | H | |
| 1.15 | $C_2H_5$ | $COOC_8H_{17}$-n | H | H | |
| 1.16 | $C_2H_5$ | $COOC_3H_7$-i | H | H | |
| 1.17 | $C_2H_5$ | $-N(CH_3)_2$ | H | H | |
| 1.18 | $C_2H_5$ | $-N(C_2H_5)_2$ | H | H | |
| 1.19 | $C_2H_5$ | $CO-N(C_2H_5)_2$ | H | H | |
| 1.20 | $C_2H_5$ | 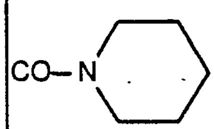 | H | H | |
| 1.21 | $C_2H_5$ | $CO-N(C_4H_9\text{-n})_2$ | H | H | |
| 1.22 | $C_2H_5$ | $CO-NHC_4H_9$-n | H | H | |
| 1.23 | $C_2H_5$ | $CO-NH-CH_2-C_6H_5$ | H | H | |
| 1.24 | $C_2H_5$ | 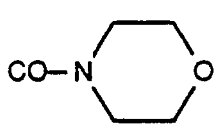 | H | H | |
| 1.25 | $C_2H_5$ | $CO-N(CH_3)-C_6H_5$ | H | H | |
| 1.26 | $C_2H_5$ | $CO-NH-C_6H_4-Cl$ | H | H | |
| 1.27 | $C_2H_5$ | $COOC_2H_5$ | H | $CH_3$ | |
| 1.28 | $C_3H_7$-i | $COOCH_3$ | H | H | $n_D^{21}$: 1.5152 |
| 1.29 | $CH_3$ | $COOC_2H_5$ | H | H | $n_D^{20}$: 1.5230 |

Tabelle 2:

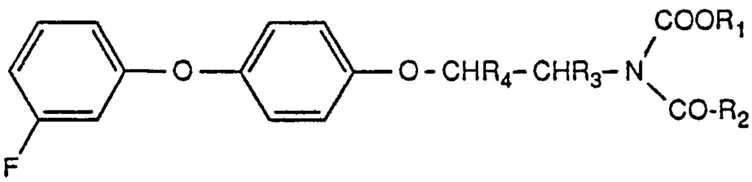

| Verb. Nr. | $R_1$ | $R_2$ | $R_3$ | $R_4$ | Physikalische Daten |
|---|---|---|---|---|---|
| 2.01 | $C_2H_5$ | $COOC_2H_5$ | H | H | $n_D^{20}$: 1.5259 |
| 2.02 | $C_2H_5$ | $COOC_4H_9$-t | H | H | |
| 2.03 | $C_2H_5$ | $COOCH_3$ | H | H | |
| 2.04 | $C_2H_5$ | $COOC_2H_5$ | $CH_3$ | H | |
| 2.05 | $CH_3$ | $COOCH_3$ | H | H | |
| 2.06 | $C_3H_7$-n | $COOC_2H_5$ | H | H | |
| 2.07 | $C_4H_9$-n | $COOCH_3$ | H | H | |
| 2.08 | $C_2H_5$ | $COOCH_3$ | $CH_3$ | H | |
| 2.09 | $C_2H_5$ | $COOCH_3$ | $CH_3$ | $CH_3$ | |
| 2.10 | $C_2H_5$ | $CH_3$ | H | H | |
| 2.11 | $C_2H_5$ | $C_3H_7$-n | H | H | |
| 2.12 | $C_2H_5$ | $OC_2H_5$ | H | H | |
| 2.13 | $C_2H_5$ | $OCH_3$ | H | H | |
| 2.14 | $C_2H_5$ | $OC_4H_9$-n | H | H | |
| 2.15 | $C_2H_5$ | $COOC_8H_{17}$-n | H | H | |
| 2.16 | $C_2H_5$ | $COOC_3H_7$-i | H | H | |
| 2.17 | $C_2H_5$ | $-N(CH_3)_2$ | H | H | |
| 2.18 | $C_2H_5$ | $-N(C_2H_5)_2$ | H | H | |
| 2.19 | $C_2H_5$ | $CO-N(C_2H_5)_2$ | H | H | |
| 2.20 | $C_2H_5$ | 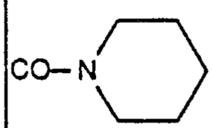 | H | H | |
| 2.21 | $C_2H_5$ | $CO-N(C_4H_9-n)_2$ | H | H | |
| 2.22 | $C_2H_5$ | $CO-NHC_4H_9$-n | H | H | |
| 2.23 | $C_2H_5$ | $CO-NH-CH_2-C_6H_5$ | H | H | |
| 2.24 | $C_2H_5$ | 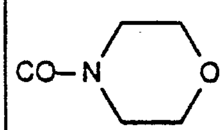 | H | H | |
| 2.25 | $C_2H_5$ | $CO-N(CH_3)-C_6H_5$ | H | H | |
| 2.26 | $C_2H_5$ | $CO-NH-C_6H_5$ | H | H | |
| 2.27 | $C_2H_5$ | $COOC_2H_5$ | H | $CH_3$ | |

Tabelle 3:

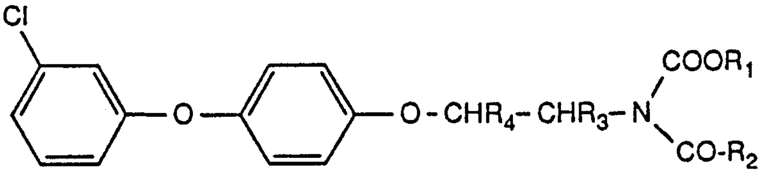

| Verb. Nr. | $R_1$ | $R_2$ | $R_3$ | $R_4$ | Physikalische Daten |
|---|---|---|---|---|---|
| 3.01 | $C_2H_5$ | $COOC_2H_5$ | H | H | $n_D^{20}$: 1.5396 |
| 3.02 | $C_2H_5$ | $COOC_4H_9$-t | H | H | |
| 3.03 | $C_2H_5$ | $COOCH_3$ | H | H | $n_D^{21}$: 1.5478 |
| 3.04 | $C_2H_5$ | $COOC_2H_5$ | $CH_3$ | H | |
| 3.05 | $CH_3$ | $COOCH_3$ | H | H | |
| 3.06 | $C_3H_7$-n | $COOC_2H_5$ | H | H | |
| 3.07 | $C_4H_9$-n | $COOCH_3$ | H | H | |
| 3.08 | $C_2H_5$ | $COOCH_3$ | $CH_3$ | H | $n_D^{20}$: 1.5443 |
| 3.09 | $C_2H_5$ | $COOCH_3$ | $CH_3$ | $CH_3$ | |
| 3.10 | $C_2H_5$ | $CH_3$ | H | H | $n_D^{22}$: 1.5542 |
| 3.11 | $C_2H_5$ | $C_3H_7$-n | H | H | $n_D^{20}$: 1.5469 |
| 3.12 | $C_2H_5$ | $OC_2H_5$ | H | H | $n_D^{24}$: 1.5426 |
| 3.13 | $C_2H_5$ | $OCH_3$ | H | H | $n_D^{20}$: 1.5493 |
| 3.14 | $C_2H_5$ | $OC_4H_9$-n | H | H | |
| 3.15 | $C_2H_5$ | $COOC_8H_{17}$-n | H | H | |
| 3.16 | $C_2H_5$ | $COOC_3H_7$-i | H | H | |
| 3.17 | $C_2H_5$ | $-N(CH_3)_2$ | H | H | $n_D^{21}$: 1.5490 |
| 3.18 | $C_2H_5$ | $-N(C_2H_5)_2$ | H | H | |
| 3.19 | $C_2H_5$ | $CO-N(C_2H_5)_2$ | H | H | $n_D^{20}$: 1.5471 |
| 3.20 | $C_2H_5$ | 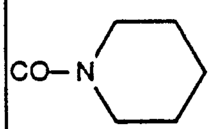 | H | H | |
| 3.21 | $C_2H_5$ | $CO-N(C_4H_9\text{-n})_2$ | H | H | $n_D^{20}$: 1.5345 |
| 3.22 | $C_2H_5$ | $CO-NHC_4H_9$-n | H | H | |
| 3.23 | $C_2H_5$ | $CO-NH-CH_2-C_6H_5$ | H | H | - |
| 3.24 | $C_2H_5$ | 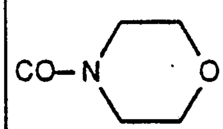 | H | H | $n_D^{21}$: 1.5569 |
| 3.25 | $C_2H_5$ | $CO-N(CH_3)-C_6H_5$ | H | H | |
| 3.26 | $C_2H_5$ | $CO-NH-C_6H_5$ | H | H | |
| 3.27 | $C_2H_5$ | $COOC_2H_5$ | H | $CH_3$ | $n_D^{20}$: 1.5303 |
| 3.28 | $C_2H_5$ | $COOC_8H_{17}$-n | H | H | $n_D^{25}$: 1.5229 |
| 3.29 | $CH_2CH=CH_2$ | $COOC_2H_5$ | H | H | $n_D^{20}$: 1.5441 |

Tabelle 4:

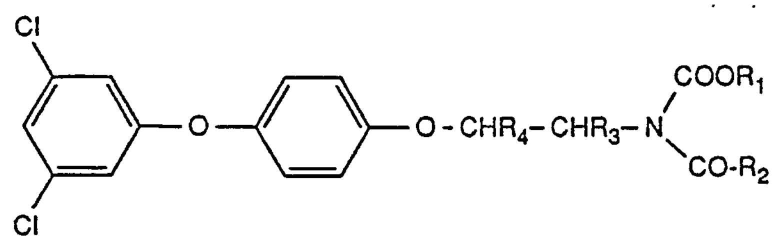

| Verb. Nr. | $R_1$ | $R_2$ | $R_3$ | $R_4$ | Physikalische Daten |
|---|---|---|---|---|---|
| 4.01 | $C_2H_5$ | $COOCH_3$ | H | H | $n_D^{20}$: 1.5512 |
| 4.02 | $C_2H_5$ | $COOC_2H_5$ | H | H | |
| 4.03 | $C_2H_5$ | $CH_3$ | H | H | |
| 4.04 | $C_2H_5$ | $C_3H_7$-n | H | H | |
| 4.05 | $C_2H_5$ | $OC_2H_5$ | H | H | |
| 4.06 | $C_2H_5$ | $N(C_2H_5)_2$ | H | H | |
| 4.07 | $C_2H_5$ | $COOC_2H_5$ | $CH_3$ | H | |
| 4.08 | $C_2H_5$ | $CH_3$ | $CH_3$ | H | |
| 4.09 | $C_2H_5$ | $COOC_2H_5$ | $CH_3$ | $CH_3$ | |

Formulierungsbeispiele für flüssige Wirkstoffe der Formel I (% = Gewichtsprozent)

| F1. Emulsions-Konzentrate | a) | b) | c) |
|---|---|---|---|
| Wirkstoff 1.05 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff Nr. 3.01 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| poxydiertes Kokosnussöl | - | - | 1 % | 5 % |
| Benzin (Siedegrenzen 160-190° C) | - | - | 94 % | - |

Die Lösungen sind zur Anwendung in Form kleinster Tropfen geeignet.

| F3. Granulate | a) | b) |
|---|---|---|
| Wirkstoff Nr. 3.10 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft

| F4. Stäubemittel | a) | b) |
|---|---|---|
| Wirkstoff Nr. 4.01 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| F5. Spritzpulver | a) | b) | c) |
|---|---|---|---|
| Wirkstoff Nr. 1.01 oder 1.03 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F6. Emulsions-Konzentrat | |
|---|---|
| Wirkstoff Nr. 1.01 oder 1.03 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff Nr. 1.01 oder 1.03 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| F8. Extruder-Granulat | |
|---|---|
| Wirkstoff Nr. 1.01 oder 1.03 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

| F9. Umhüllungs-Granulat | |
|---|---|
| Wirkstoff Nr. 1.03 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| F10. Suspensions-Konzentrat | |
|---|---|
| Wirkstoff Nr. 1.03 | 40 % |
| Aethylenglylol | 10 % |
| Nonylphenolpolyäthylenglykoläther (15 Mol AeO) | 6 % |
| Na-Ligninsulfonat | 10 % |
| Carboxymethylcellulose | 1 % |
| 37%-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

In den nachfolgenden biologischen Beispielen bedeutet eine gute Wirkung, dass der erwünschte Effekt zu mindestens 50 bis 60 % eintritt.

Beispiel B1: Wirkung gegen Dermanyssus gallinae

In einem nach oben offenen Glasbehälter werden 2 bis 3 ml einer 10 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen

werden kann. Nach 3 Tagen wird die Mortalität der Milben durch Auszählen der toten Individuen ermittelt und in Prozent angegeben.

Verbindungen der Tabellen 1, 2, 3, 4 zeigen eine gute Wirkung gegen Dermanyssus gallinae.

Beispiel B2: Wirkung gegen Boophilus microplus

Vollgesogene adulte Zecken-Weibchen werden auf eine PVC-Platte aufgeklebt und mit einem Wattebausch überdeckt. Zur Behandlung werden die Testtiere mit 10 ml einer wässrigen Testlösung, die 125 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.

Verbindungen gemäss Tabellen 1, 2, 3, 4 zeigen in diesem Test gute Wirkung gegen Boophilus microplus. Insbesondere die Verbindungen 1.01, 1.03, 3.01, 3.10, 3.12 und 3.17 zeigen eine Wirkung über 80 %.

Beispiel B3: Ovizide Wirkung auf Cydia pomonella

Auf Filterpapier abgelegte Eier von Cydia pomonella werden für eine kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Verbindungen gemäss Tabelle 1 zeigen in diesem Test gute Wirkung gegen Cydia pomonella. Insbesondere die Verbindungen 1.01, 1.03, 1.05, 3.01, 3.03, 3.12, 3.13 und 3.17 zeigen eine Wirkung über 80 %.

Beispiel B4: Ovizide Wirkung auf Adoxophyes reticulana

Auf Filterpapier abgelegte Eier von Adoxophyes reticulana werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion.

Verbindungen gemäss Tabellen 1, 2, 3, 4 zeigen in diesem Test gute Wirkung gegen Adoxophyes reticulana. Insbesondere die Verbindungen 1.01, 1.03, 1.05, 3.01, 3.03, 3.12, 3.13 und 3.17 zeigen eine Wirkung über 80 %.

Beispiel B5: Ovizide Wirkung auf Lobesia botrana

Auf Filterpapier abgelegte Eier von Lobesia botrana werden für kurze Zeit in eine acetonisch-wässrige Tesllösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Verbindungen gemäss Tabellen 1, 2, 3, 4 zeigen in diesem Test gute Wirkung gegen Lobesia botrana. Insbesondere die Verbindungen 1.01 und 1.03 zeigen eine Wirkung über 80%.

Beispiel B6: Ovizide Wirkung auf Heliothis virescens

Auf Filterpapier abgelegte Eier von Heliothis virescens werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Verbindungen gemäss Tabellen 1, 2, 3, 4 zeigen in diesem Test gute Wirkung gegen Heliothis virescens. Insbesondere die Verbindungen 1.01, 1.03 und 3.10 zeigen eine Wirkung über 80%.

Beispiel B7: Wirkung gegen Aonidiella aurantii

Kartoffelknollen werden mit Wanderlarven ("Crawlern") von Aonidiella aurantii (rote Citrus-Schildlaus) besiedelt. Nach etwa 2 Wochen werden die Kartoffeln in eine wässrige Emulsions- resp. Suspensions-Spritzbrühe getaucht, die den zu prüfenden Wirkstoff in einer Konzentration von 400 ppm enthält. Nach dem Abtrocknen der so behandelten Kartoffelknollen werden diese in einem Plastikbehälter inkubiert. Zur Auswertung wird 10-12 Wochen später die Ueberlebensrate der Wanderlarven der ersten Folgegeneration der behandelten Schildlaus-Population mit derjenigen der unbehandelten Kontrollansätze verglichen.

Verbindungen gemäss Tabellen 1, 2, 3, 4 zeigen in diesem Test gute Wirkung gegen Aonidiella aurantii.

Beispiel B8: Wirkung gegen Nilaparvata lugens

Reispflanzen werden mit einer wässrigen Emulsions-Spritzbrühe, die 400 ppm des Wirkstoffes enthält, behandelt Nach dem Antrocknen des Spritzbelages werden die Reis pflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen der Tabellen 1,2,3 und 4 zeigen eine gute Wirkung gegen Nilaparvata lugens in diesem Test. Insbesondere die Verbindungen 1.01, 1.03 1.05, 3.01, 3.03, 3.10, 3.11, 3.12, 3.13 und 3.17 zeigen eine Wirkung über 80 %.

Beispiel B9: Wirkung gegen Nephotettix cincticeps

Reispflanzen werden mit einer wässrigen Emulsions-Spritzbrühe, die 400 ppm des Wirkstoffes enthält, behandelt Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen der Tabellen 1, 2, 3 und 4 zeigen eine gute Wirkung gegen Nephotettix cincticeps in diesem Test. Insbesondere die Verbindungen 1.01, 1.03 1.05, 3.01, 3.03, 3.10, 3.11, 3.12, 3.13 und 3.17 zeigen eine Wirkung über 80%.

Beispiel B10: Wirkung gegen Bemisia tabaci

Buschbohnen-Pflanzen werden in Gazekäfige gestellt und mit Adulten von Bemisia tabaci (Weiss Fliege) besiedelt. Nach erfolgter Eiablage werden alle Adulten entfernt und 10 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu den unbehandelten Kontrollansätzen.

Die Verbindungen der Tabellen 1, 2, 3 und 4 zeigen gute Wirkung in diesem Test. Die Verbindungen 1.01, 1.03, 1.05 und 3.03 sind bei Konzentrationen von 10 ppm noch zu 80 % wirksam.

BeispieL B11: Ovo/larvizide Wirkung auf Heliothis virescens

Auf Baumwolle abgelegte Eier von Heliothis virescens werden mit einer wässrigen Emulsions-Spritzbrühe, die 400 ppm des Wirkstoffes enthält, besprüht. Nach 8 Tagen wird der prozentuale Schlupf der Eier und die Ueberlebensrate der Raupen im Vergleich zu unbehandelten Kontrollansätzen ausgewertet (% Reduktion der Population).

Verbindungen gemäss Tabellen 1, 2, 3 und 4 zeigen in diesem Test gute Wirkung gegen Heliothis virescens. Insbesondere die Verbindungen 1.01, 1.03, 1.05, 3.01, 3.03, 3.11, 3.13 und 3.17 zeigen eine Wirkung über 80 %.

**Ansprüche**

1. N-Acyl-2-[4-(halogenphenoxy)phenoxy]-äthylcarbaminsäureester der Formel I

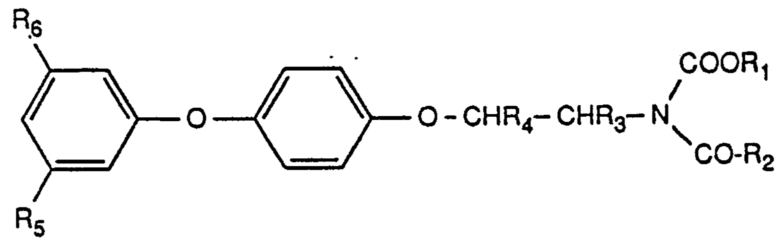

(I)

worin
$R_1$ $C_1$-$C_8$-Alkyl oder $C_3$-$C_5$-Alkenyl,
$R_2$ $C_1$-$C_8$-Alkyl, $C_1$-$C_8$-Alkoxy, -CO-$R_7$ oder -$NR_8R_9$,
$R_3$ und $R_4$ unabhängig voneinander für Wasserstoff oder Methyl,
$R_5$ für Chlor oder Fluor,
$R_6$ entweder für den gleichen Substituenten wie $R_5$ oder für Wasserstoff,
$R_7$ $C_1$-$C_8$-Alkoxy oder -$NR_{10}R_{11}$,
$R_8$ für $C_1$-$C_4$-Alkyl,
$R_9$ für $C_1$-$C_4$-Alkyl oder
$R_8$ und $R_9$ zusammen für eine $C_4$-$C_6$-Alkylenkette, welche durch Sauerstoff, Schwefel oder -$NCH_3$- unterbrochen sein kann,
$R_{10}$ für Wasserstoff oder $C_1$-$C_4$-Alkyl und
$R_{11}$ für Wasserstoff oder $C_1$-$C_4$-Alkyl, Benzyl, Phenyl oder durch Halogen oder Methyl substituiertes Phenyl oder
$R_{10}$ und $R_{11}$ zusammen für eine $C_4$-$C_6$-Alkylenkette, welche durch Sauerstoff, Schwefel oder -$NCH_3$- unterbrochen sein kann,
stehen.

2.Verbindungen gemäss Anspruch 1, dadurch gekennzeichnet, dass $R_1$ für $C_1$-$C_4$-Alkyl, $R_2$ für $C_1$-$C_4$-Alkyl, $C_1$-$C_4$-Alkoxy, $C_1$-$C_4$-Alkoxycarbonyl, $C_1$-$C_4$-Dialkylamino oder -$CONR_{10}R_{11}$ und $R_5$ und $R_6$ beide entweder für Fluor oder für Chlor oder $R_5$ für Chlor oder Fluor und $R_6$ für Wasserstoff, stehen, wobei $R_{10}$ Wasserstoff oder $C_1$-$C_4$-Alkyl, $R_{11}$ Wasserstoff, $C_1$-$C_4$-Alkyl oder Phenyl oder $R_{10}$ und $R_{11}$ zusammen eine $C_4$-$C_6$-Alkylenkette, die durch Sauerstoff unterbrochen sein kann, bedeuten.

3. Verbindungen gemäss Anspruch 2, dadurch gekennzeichnet, dass $R_2$ für $C_1$-$C_4$-Alkyl, $C_1$-$C_4$-Alkoxy, $C_1$-$C_4$-Alkoxycarbonyl, $C_1$-$C_2$-Dialkylamino, Di-($C_1$-$C_4$-alkyl)-aminocarbonyl oder $C_4$-$C_6$-Alkylenaminocarbonyl steht.

4. Verbindungen gemäss Anspruch 1, dadurch gekennzeichnet, dass $R_1$ für $C_1$-$C_4$-Alkyl, $R_2$ für $C_1$-$C_4$-Alkyl, $C_1$-$C_4$-Alkoxy, Di-($C_1$-$C_2$-alkyl)-aminocarbonyl oder $C_1$-$C_4$-Alkoxycarbonyl, $R_3$ für Methyl oder Wasserstoff, $R_4$ für Wasserstoff und $R_5$ und $R_6$ für Fluor oder Chlor stehen.

5. Verbindungen gemäss Anspruch 1 ausgewählt aus der Gruppe

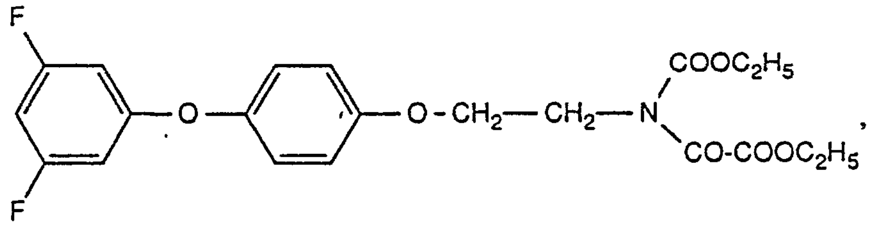

,

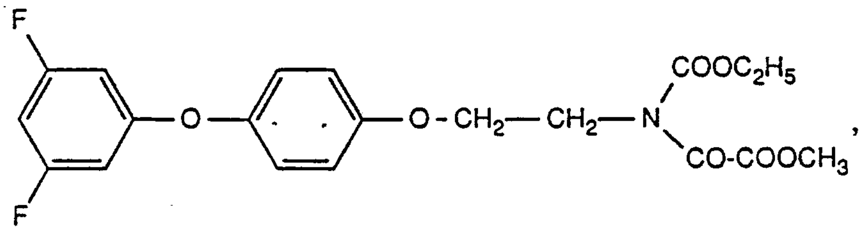

,

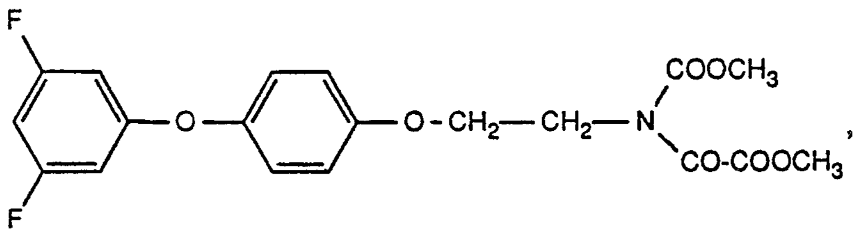

,

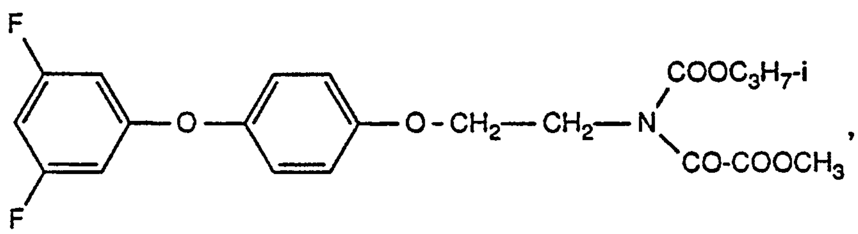

,

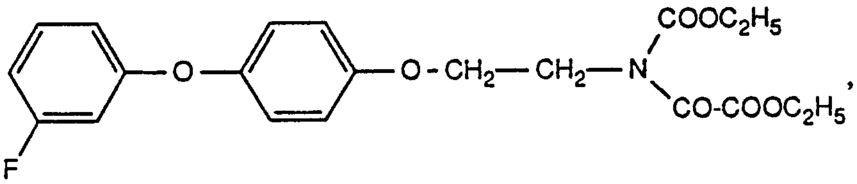
,

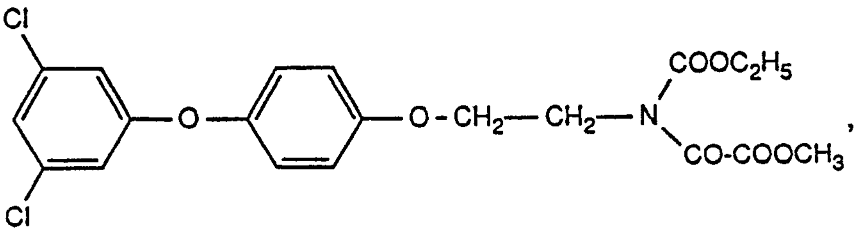
,

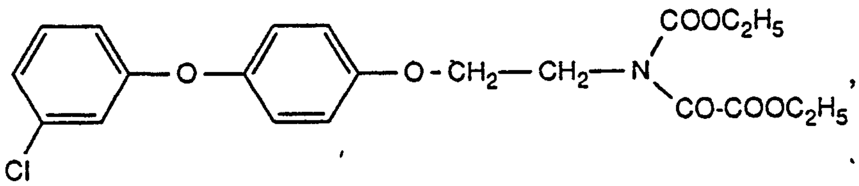
,

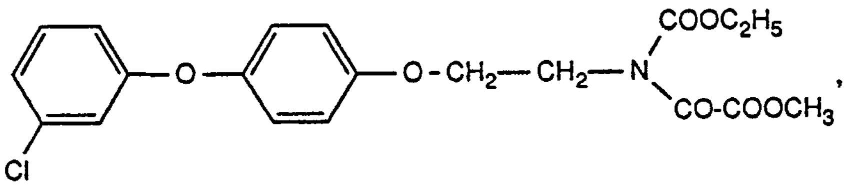
,

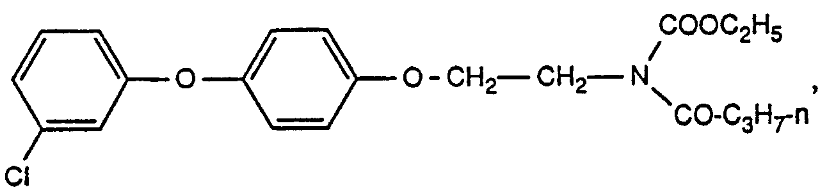
,

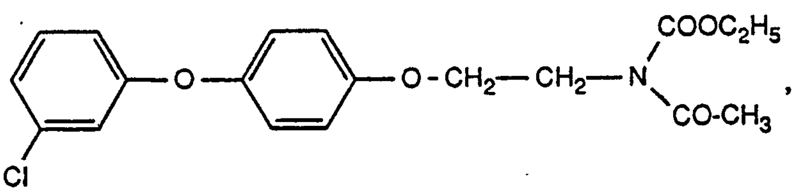
,

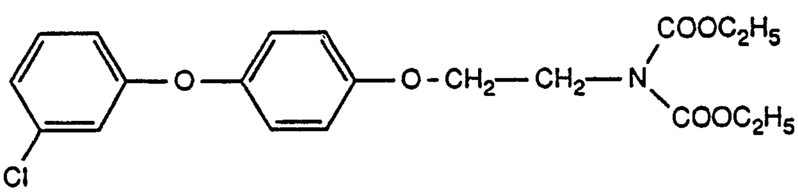
,

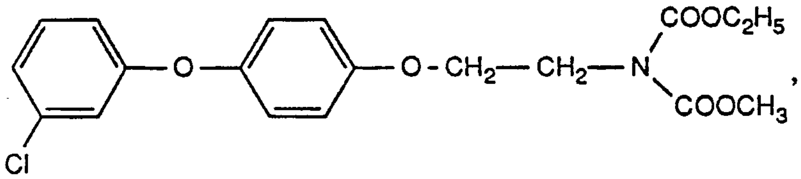
,

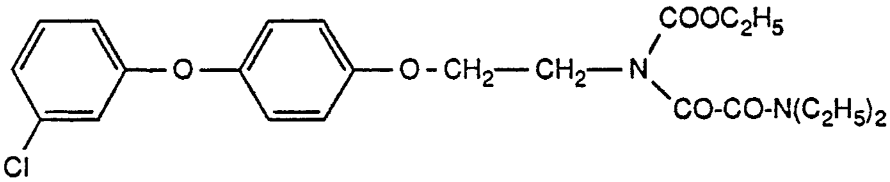
,

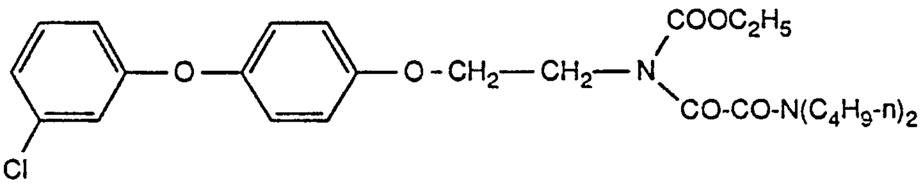
,

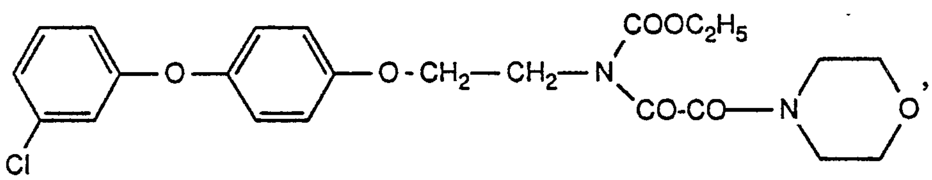
,

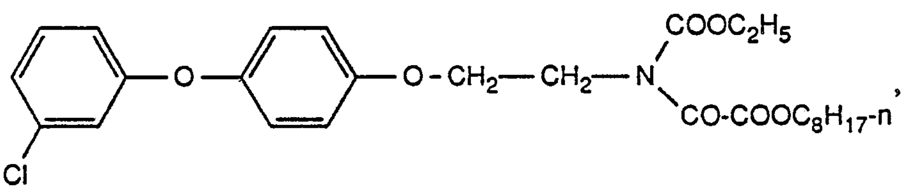
,

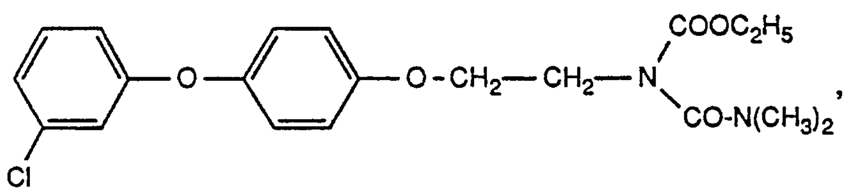
,

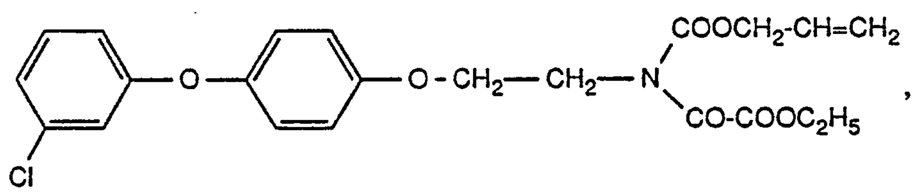
,

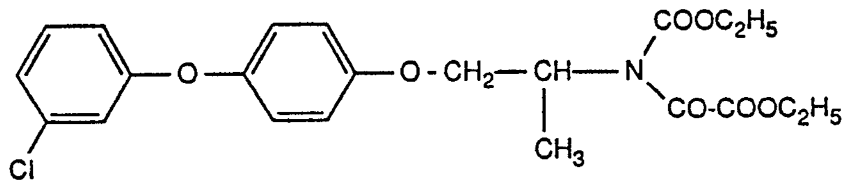

und

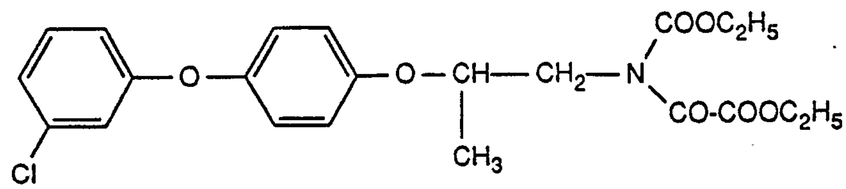

6. Verfahren zur Herstellung der Verbindungen der Formel I gemäss Anspruch 1, dadurch gekennzeichnet, dass man einen Halogenphenoxyphenoxyäthylcarbaminsäureester der Formel II

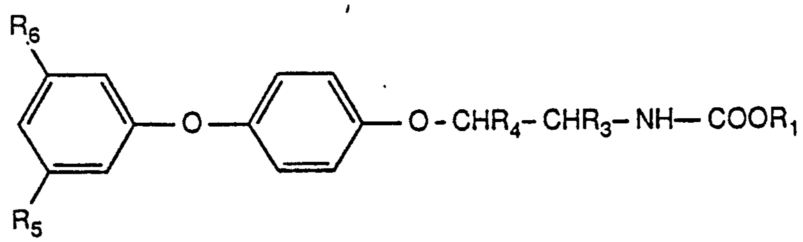

(II)

worin $R_1$, $R_3$, $R_4$, $R_5$ und $R_6$ die unter Formel I gegebenen Bedeutungen haben, mit einer Acylverbindung der Formel III

X-CO-$R_2$ (III)

worin $R_2$ die unter Formel I gegebene Bedeutung hat und X für Chlor, Brom, -O-CO-$R_2$ oder -OCO($C_1$-$C_4$-Alkyl) steht, acyliert.

7. Verfahren gemäss Anspruch 6, dadurch gekennzeichnet, dass man die Acylierung in Gegenwart einer Base durchführt.

8. Schädlingsbekämpfungsmittel, welches als aktive Komponente mindestens eine Verbindung der Formel I

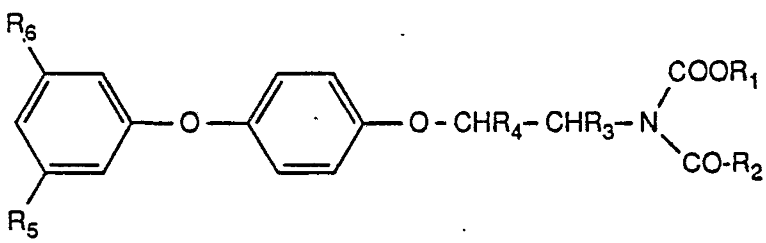

(I)

worin

$R_1$ $C_1$-$C_8$-Alkyl oder $C_3$-$C_5$-Alkenyl,

$R_2$ $C_1$-$C_8$-Alkyl, $C_1$-$C_8$-Alkoxy, -CO-$R_7$ oder -$NR_8R_9$,

$R_3$ und $R_4$ unabhängig voneinander für Wasserstoff oder Methyl,

$R_5$ für Chlor oder Fluor,

$R_6$ entweder für den gleichen Substituenten wie $R_5$ oder für Wasserstoff,

$R_7$ $C_1$-$C_8$-Alkoxy oder -$NR_{10}R_{11}$,

$R_8$ für $C_1$-$C_4$-Alkyl,

$R_9$ für $C_1$-$C_4$-Alkyl oder

$R_8$ und $R_9$ zusammen für eine $C_4$-$C_6$-Alkylenkette, welche durch Sauerstoff, Schwefel oder -$NCH_3$-

unterbrochen sein kann,
$R_{10}$ für Wasserstoff oder $C_1$-$C_4$-Alkyl und
$R_{11}$ für Wasserstoff oder $C_1$-$C_4$-Alkyl, Benzyl, Phenyl oder durch Halogen oder Methyl substituiertes Phenyl oder
$R_{10}$ und $R_{11}$ zusammen für eine $C_4$-$C_6$-Alkylenkette, welche durch Sauerstoff, Schwefel oder $-NCH_3$- unterbrochen sein kann, stehen, enthält.

9. Mittel gemäss Anspruch 8, dadurch gekennzeichnet, dass es zusätzlich mindestens noch einen Träger enthält.

10.Verwendung einer Verbindung der Formel I

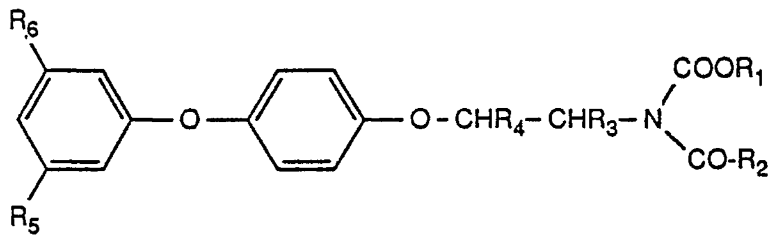

(I)

worin
$R_1$ $C_1$-$C_8$-Alkyl oder $C_3$-$C_5$-Alkenyl,
$R_2$ $C_1$-$C_8$-Alkyl, $C_1$-$C_8$-Alkoxy, $-CO-R_7$ oder $-NR_8R_9$,
$R_3$ und $R_4$ unabhängig voneinander für Wasserstoff oder Methyl,
$R_5$ für Chlor oder Fluor,
$R_6$ entweder für den gleichen Substituenten wie $R_5$ oder für Wasserstoff,
$R_7$ $C_1$-$C_8$-Alkoxy oder $-NR_{10}R_{11}$,
$R_8$ für $C_1$-$C_4$-Alkyl,
$R_9$ für $C_1$-$C_4$-Alkyl oder
$R_8$ und $R_9$ zusammen für eine $C_4$-$C_6$-Alkylenkette, welche durch Sauerstoff, Schwefel oder $-NCH_3$- unterbrochen sein kann,
$R_{10}$ für Wasserstoff oder $C_1$-$C_4$-Alkyl und
$R_{11}$ für Wasserstoff oder $C_1$-$C_4$-Alkyl, Benzyl, Phenyl oder durch Halogen oder Methyl substituiertes Phenyl oder
$R_{10}$ und $R_{11}$ zusammen für eine $C_4$-$C_6$-Alkylenkette, welche durch Sauerstoff, Schwefel oder $-NCH_3$- unterbrochen sein kann, stehen,
zur Bekämpfung von Schädlingen an Pflanzen und Tieren und im Haushalt.

11. Verwendung gemäss Anspruch 10 zur Bekämpfung von Arthropoden, insbesondere von Insekten und Arachniden.

12. Verwendung gemäss Anspruch 11 zur Bekämpfung von Larven und Eiern phytophager Schadinsekten und -milben.

13. Verwendung gemäss Anspruch 11 als Chemosterilisierungsmittel und Ovizid zur Bekämpfung pflanzenschädigender Insekten und Milben.

14. Das Zwischenprodukt der Formel IVa

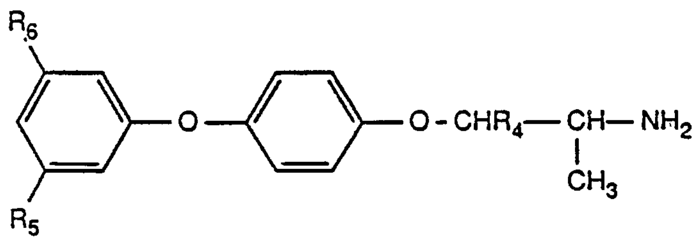

(IVa)

worin
$R_4$ Wasserstoff oder Methyl und
$R_5$ Chlor oder Fluor bedeuten und

$R_6$ entweder die gleiche Bedeutung wie $R_5$ hat oder für Wasserstoff steht.

15. Verfahren zur Herstellung der Verbindung IVa gemäss Anspruch 14, dadurch gekennzeichnet, dass man ein 4-(Halogenphenoxy)-phenol der Formel

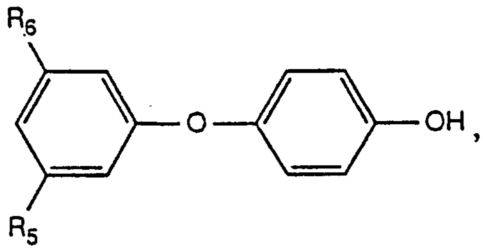

worin $R_5$ und $R_6$ die unter Formel I in Anspruch 1 gegebenen Bedeutungen haben, mit einem Chlor- oder Bromketon der Formel

$Hal\text{-}CHR_4\text{-}CO\text{-}CH_3$ ,

worin Hal Chlor oder Brom und $R_4$ Wasserstoff oder Methyl bedeuten, in Gegenwart von Kaliumcarbonat umsetzt und das so erhaltene 4-(Halogenphenoxy)-phenoxyaceton-Derivat der Formel

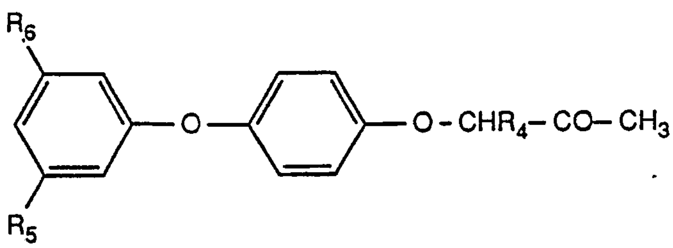

in Gegenwart von Ammoniak, Wasserstoff und Raney-Nickel unter Druck erhitzt.

16. Verfahren zur Herstellung der Verbindungen der Formel I, worin $R_2$ für die Gruppe $\text{-CO-}R_7$ steht, dadurch gekennzeichnet, dass man den Carbaminsäureester der Formel II gemäss Anspruch 6 zunächst mit Oxalsäuredichlorid umsetzt und das erhaltene Zwischenprodukt der Formel

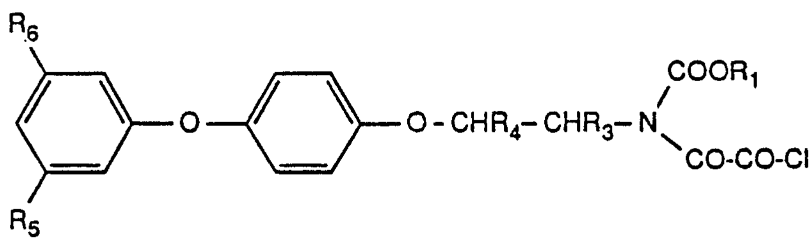

worin $R_1$, $R_3$, $R_4$, $R_5$ und $R_6$ die unter Formel I gegebenen Bedeutungen haben, in Gegenwart einer Base mit einem Alkohol oder einem Amin der Formel

$H\text{-}R_7$

worin $R_7$ die unter Formel I gegebene Bedeutung hat, umsetzt.

Patentansprüche für folgenden Vertragsstaat: ES

1. Schädlingsbekämpfungsmittel, welches als aktive Komponente mindestens einen N-Acyl-2-[4-(halogenphenoxy)phenoxy]-äthylcarbaminsäureester der Formel I

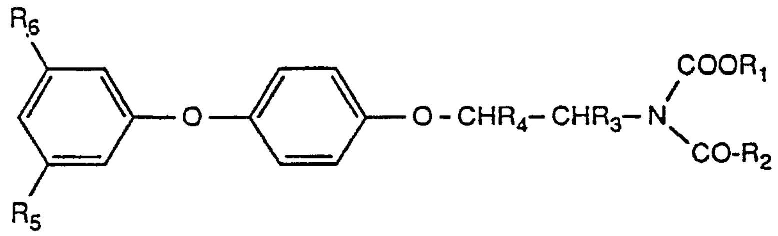

(I)

worin
$R_1$ $C_1$-$C_8$-Alkyl oder $C_3$-$C_5$-Alkenyl,
$R_2$ $C_1$-$C_8$-Alkyl, $C_1$-$C_8$-Alkoxy, -CO-$R_7$ oder -$NR_8R_9$,
$R_3$ und $R_4$ unabhängig voneinander für Wasserstoff oder Methyl,
$R_5$ für Chlor oder Fluor,
$R_6$ entweder für den gleichen Substituenten wie $R_5$ oder für Wasserstoff,
$R_7$ $C_1$-$C_8$-Alkoxy oder -$NR_{10}R_{11}$,
$R_8$ für $C_1$-$C_4$-Alkyl,
$R_9$ für $C_1$-$C_4$-Alkyl oder
$R_8$ und $R_9$ zusammen für eine $C_4$-$C_6$-Alkylenkette, welche durch Sauerstoff, Schwefel oder -$NCH_3$- unterbrochen sein kann,
$R_{10}$ für Wasserstoff oder $C_1$-$C_4$-Alkyl und
$R_{11}$ für Wasserstoff oder $C_1$-$C_4$-Alkyl, Benzyl, Phenyl oder durch Halogen oder Methyl substituiertes Phenyl oder
$R_{10}$ und $R_{11}$ zusammen für eine $C_4$-$C_6$-Alkylenkette, welche durch Sauerstoff, Schwefel oder -$NCH_3$- unterbrochen sein kann,
stehen, enthält.

2. Mittel gemäss Anspruch 1, dadurch gekennzeichnet, dass $R_1$ für $C_1$-$C_4$-Alkyl, $R_2$ für $C_1$-$C_4$-Alkyl, $C_1$-$C_4$-Alkoxy, $C_1$-$C_4$-Alkoxycarbonyl, $C_1$-$C_4$-Dialkylamino oder -$CONR_{10}R_{11}$ und $R_5$ und $R_6$ beide entweder für Fluor oder für Chlor oder $R_5$ für Chlor oder Fluor und $R_6$ für Wasserstoff, stehen, wobei $R_{10}$ Wasserstoff oder $C_1$-$C_4$-Alkyl, $R_{11}$ Wasserstoff, $C_1$-$C_4$-Alkyl oder Phenyl oder $R_{10}$ und $R_{11}$ zusammen eine $C_4$-$C_6$-Alkylenkette, die durch Sauerstoff unterbrochen sein kann, bedeuten.

3. Mittel gemäss Anspruch 2, dadurch gekennzeichnet, dass $R_2$ für $C_1$-$C_4$-Alkyl, $C_1$-$C_4$-Alkoxy, $C_1$-$C_4$-Alkoxycarbonyl, $C_1$-$C_2$-Dialkylamino, Di-($C_1$-$C_4$-alkyl)-aminocarbonyl oder $C_4$-$C_6$-Alkylenaminocarbonyl steht.

4. Mittel gemäss Anspruch 1, dadurch gekennzeichnet, dass $R_1$ für $C_1$-$C_4$-Alkyl, $R_2$ für $C_1$-$C_4$-Alkyl, $C_1$-$C_4$-Alkoxy, Di-($C_1$-$C_2$-alkyl)-aminocarbonyl oder $C_1$-$C_4$-Alkoxycarbonyl, $R_3$ für Methyl oder Wasserstoff, $R_4$ für Wasserstoff und $R_5$ und $R_6$ für Fluor oder Chlor stehen.

5. Mittel gemäss Anspruch 1 enthaltend einen Wirkstoff ausgewählt aus der Gruppe

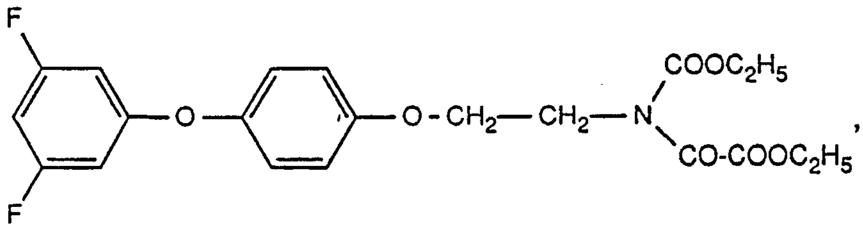

,

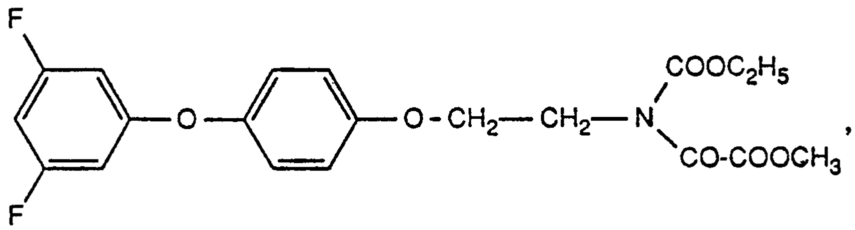

,

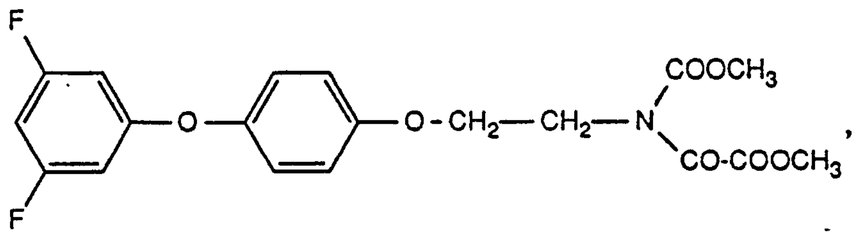

,

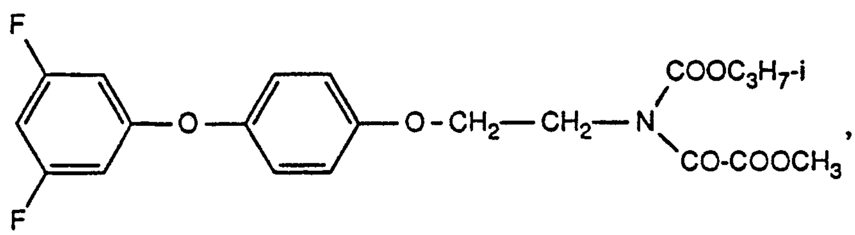

,

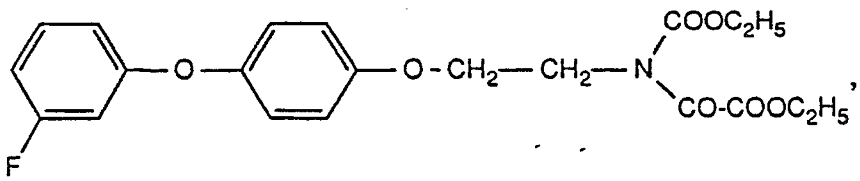

,

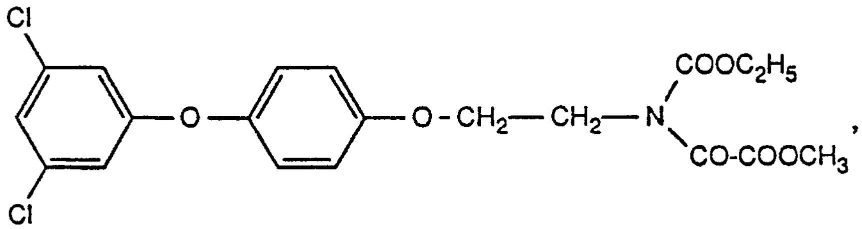

,

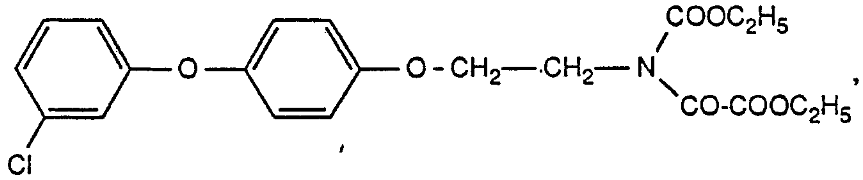

,

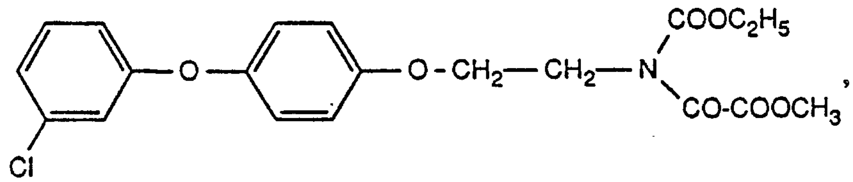

,

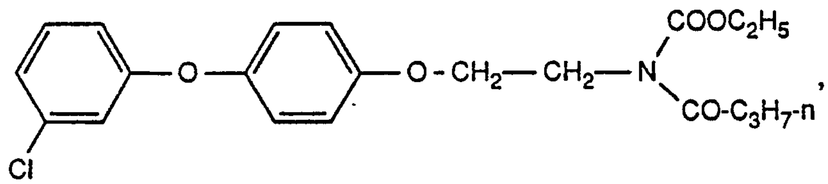

,

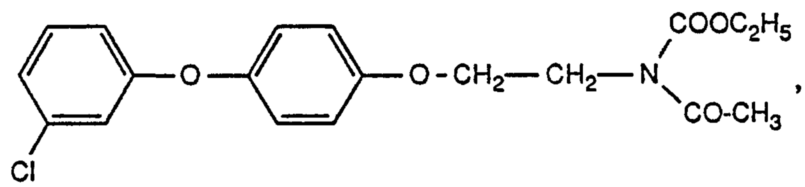

,

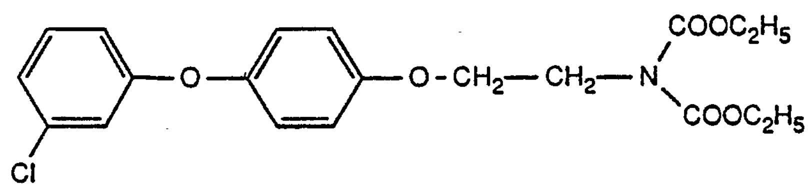

,

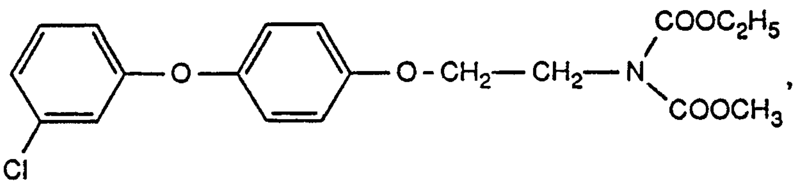

,

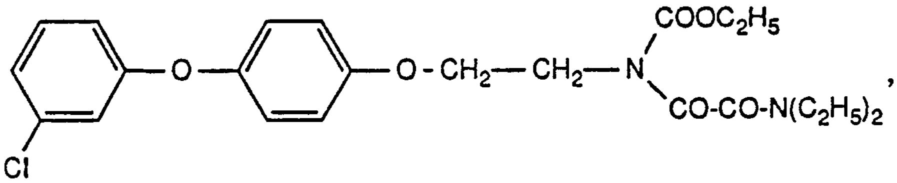

,

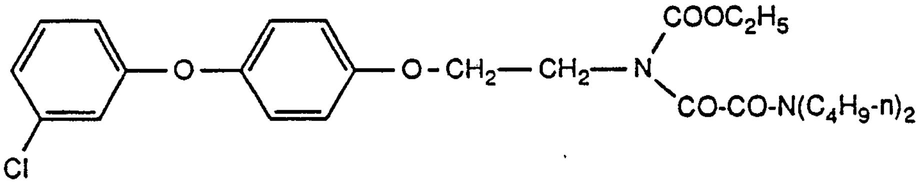

,

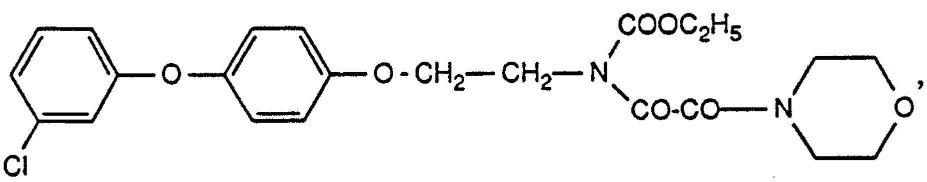

,

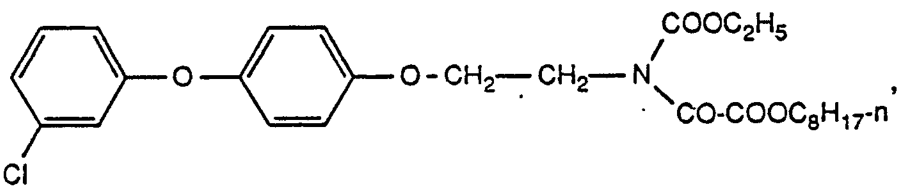

,

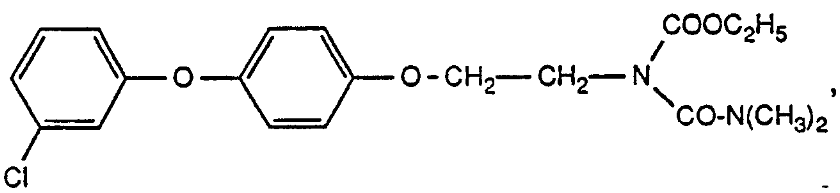

,

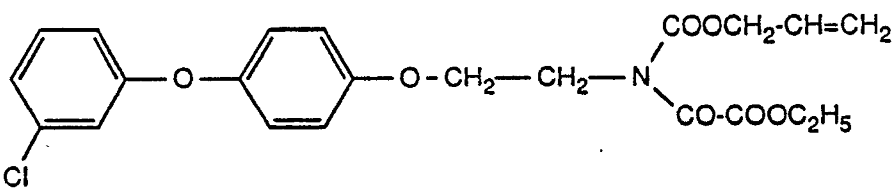

,

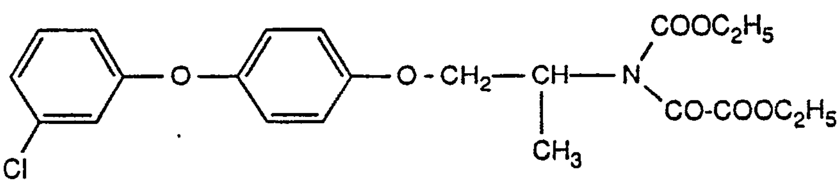

und

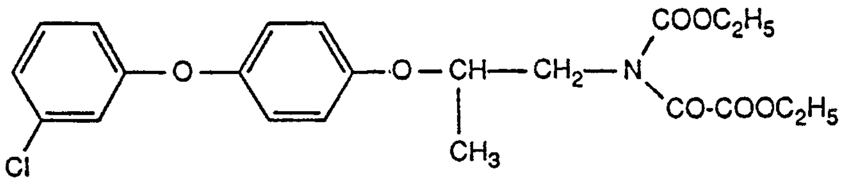

.

6. Verfahren zur Herstellung der Verbindungen der Formel I gemäss Anspruch 1, dadurch gekennzeichnet, dass man einen Halogenphenoxyphenoxyäthylcarbaminsäureester der Formel II

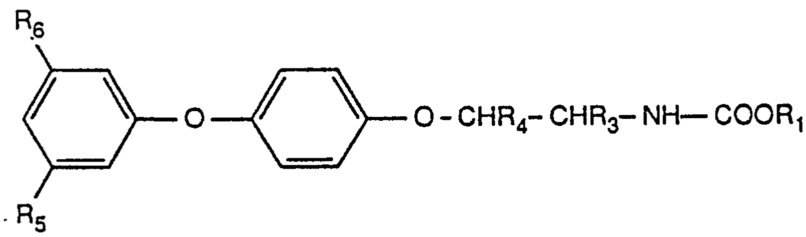

(II)

worin $R_1$, $R_3$, $R_4$, $R_5$ und $R_6$ die unter Formel I gegebenen Bedeutungen haben, mit einer Acylverbindung der Formel III

$X\text{-}CO\text{-}R_2$ (III)

worin $R_2$ die unter Formel I gegebene Bedeutung hat und X für Chlor, Brom, $-O-CO-R_2$ oder $-OCO(C_1\text{-}C_4\text{-}Alkyl)$ steht, acyliert.

7. Verfahren gemäss Anspruch 6, dadurch gekennzeichnet, dass man die Acylierung in Gegenwart einer Base durchführt.

8. Mittel gemäss Anspruch 1, dadurch gekennzeichnet, dass es zusätzlich mindestens noch einen Träger enthält.

9. Verwendung einer Verbindung der Formel I

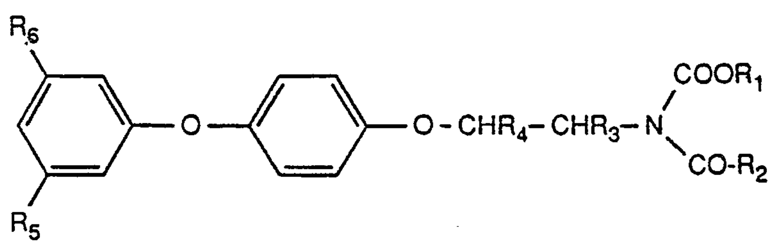

(I)

worin

$R_1$ $C_1$-$C_8$-Alkyl oder $C_3$-$C_5$-Alkenyl,

$R_2$ $C_1$-$C_8$-Alkyl, $C_1$-$C_8$-Alkoxy, $-CO-R_7$ oder $-NR_8R_9$,

$R_3$ und $R_4$ unabhängig voneinander für Wasserstoff oder Methyl,

$R_5$ für Chlor oder Fluor,

$R_6$ entweder für den gleichen Substituenten wie $R_5$ oder für Wasserstoff,

$R_7$ $C_1$-$C_8$-Alkoxy oder $-NR_{10}R_{11}$,

$R_8$ für $C_1$-$C_4$-Alkyl,

$R_9$ für $C_1$-$C_4$-Alkyl oder

$R_8$ und $R_9$ zusammen für eine $C_4$-$C_6$-Alkylenkette, welche durch Sauerstoff, Schwefel oder $-NCH_3$-unterbrochen sein kann,
$R_{10}$ für Wasserstoff oder $C_1$-$C_4$-Alkyl und
$R_{11}$ für Wasserstoff oder $C_1$-$C_4$-Alkyl, Benzyl, Phenyl oder durch Halogen oder Methyl substituiertes Phenyl oder
$R_{10}$ und $R_{11}$ zusammen für eine $C_4$-$C_6$-Alkylenkette, welche durch Sauerstoff, Schwefel oder $-NCH_3$-unterbrochen sein kann, stehen,
zur Bekämpfung von Schädlingen an Pflanzen und Tieren und im Haushalt.

10. Verwendung gemäss Anspruch 9 zur Bekämpfung von Arthropoden, insbesondere von Insekten und Arachniden.

11. Verwendung gemäss Anspruch 10 zur Bekämpfung von Larven und Eiern phytophager Schadinsekten und -milben.

12. Verwendung gemäss Anspruch 10 als Chemosterilisierungsmittel und Ovizid zur Bekämpfung pflanzenschädigender Insekten und Milben.

13. Verfahren zur Herstellung der Zwischenprodukte der Formel IVa

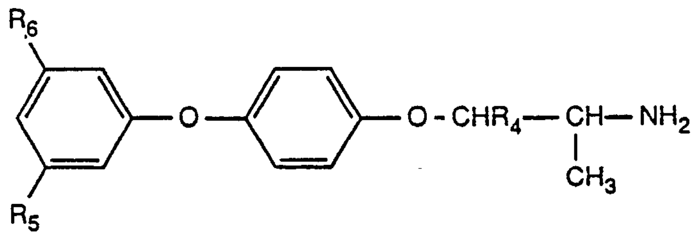

(IVa)

worin
$R_4$ Wasserstoff oder Methyl und
$R_5$ Chlor oder Fluor bedeuten und
$R_6$ entweder die gleiche Bedeutung wie $R_5$ hat oder für Wasserstoff steht, dadurch gekennzeichnet, dass man ein 4-(Halogenphenoxy)-phenol der Formel

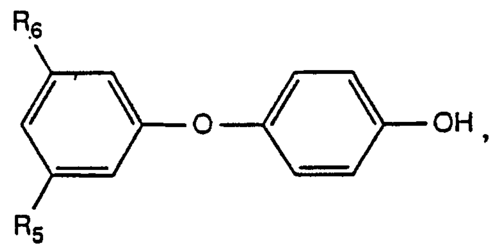

worin $R_5$ und $R_6$ die unter Formel I in Anspruch 1 gegebenen Bedeutungen haben, mit einem Chlor- oder Bromketon der Formel
$Hal-CHR_4-CO-CH_3$ ,
worin Hal Chlor oder Brom und $R_4$ Wasserstoff oder Methyl bedeuten, in Gegenwart von Kaliumcarbonat umsetzt und das so erhaltene 4-(Halogenphenoxy)-phenoxyaceton-Derivat der Formel

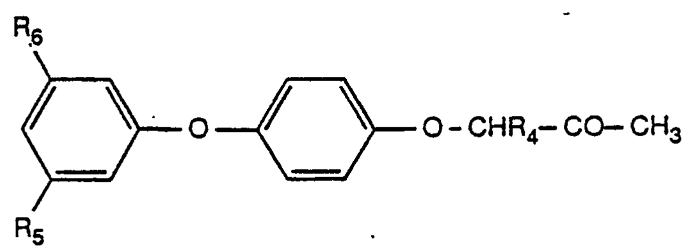

in Gegenwart von Ammoniak, Wasserstoff und Raney-Nickel unter Druck erhitzt.

14. Verfahren zur Herstellung der Verbindungen der Formel I, worin $R_2$ für die Gruppe $-CO-R_7$ steht, dadurch gekennzeichnet, dass man den Carbaminsäureester der Formel II gemäss Anspruch 6 zunächst mit Oxalsäuredichlorid umsetzt und das erhaltene Zwischenprodukt der Formel

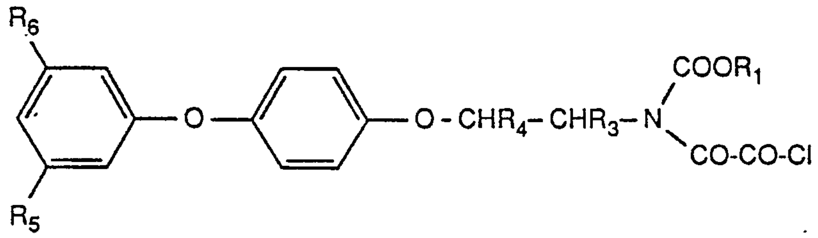

worin $R_1$, $R_3$, $R_4$, $R_5$ und $R_6$ die unter Formel I gegebenen Bedeutungen haben, in Gegenwart einer Base mit einem Alkohol oder einem Amin der Formel

$H-R_7$

worin $R_7$ die unter Formel I gegebene Bedeutung hat, umsetzt.